# FASCICULE DE BREVET EUROPEEN

(11) **EP 2 542 688 B1**
(45) Date de publication et mention de la délivrance du brevet: **11.05.2016**
(21) Numéro de dépôt: 11712939.5
(22) Date de dépôt: 01.03.2011
(51) Int. Cl.: C12Q 1/04, C12Q 1/34, C12Q 1/40

(54) **PROCÉDÉ RAPIDE DE DÉTECTION D'ENZYMES ET DE MICROORGANISMES**
SCHNELLES VERFAHREN ZUM NACHWEIS VON ENZYMEN UND MIKROORGANISMEN
QUICK METHOD FOR DETECTING ENZYMES AND MICRO-ORGANISMS

(30) Priorité: 01.03.2010 FR 1051441
(43) Date de publication de la demande: 09.01.2013
(60) Demande divisionnaire: 16153038.1
(73) Titulaire: Bio-Rad Innovations, 92430 Marnes-La-Coquette (FR)
(72) Inventeur: DALLENNE, Caroline, 59184 Sainghin-en-Weppes (FR); FAVIER, Christine, 59470 Wormhout (FR)
(74) Mandataire: Domenego, Bertrand
(86) Numéro de dépôt international: PCT/FR2011/050417
(87) Numéro de publication internationale: WO 2011/107703

(56) Documents cités:
- WO-A1-2009/051838
- WO-A2-2010/016911
- FR-A1- 2 882 370
- CHEN JIUNN-RONG ET AL: "Rapid identification and susceptibility testing using the VITEK (R) 2 system using culture fluids from positive BacT/ALERT (R) blood cultures", CHINESE JOURNAL OF MICROBIOLOGY. ZHONGHUA MINGUO, LIPPINCOTT WILLIAMS & WILKINS ASIA, HK, vol. 41, no. 3, 1 juin 2008 (2008-06-01), pages 259-264, XP009142065, ISSN: 0253-2662 cité dans la demande
- LUPETTI A ET AL: "Rapid identification and antimicrobial susceptibility profiling of Gram-positive cocci in blood cultures with the Vitek 2 system", EUROPEAN JOURNAL OF CLINICAL MICROBIOLOGY & INFECTIOUS DISEASES, SPRINGER, BERLIN, DE, vol. 29, no. 1, 10 novembre 2009 (2009-11-10), pages 89-95, XP019763658, ISSN: 1435-4373
- LUPETTI A ET AL: "Rapid identification and antimicrobial susceptibility testing of Gram-positive cocci in blood cultures by direct inoculation into the BD Phoenix system.", CLINICAL MICROBIOLOGY AND INFECTION : THE OFFICIAL PUBLICATION OF THE EUROPEAN SOCIETY OF CLINICAL MICROBIOLOGY AND INFECTIOUS DISEASES JUL 2010 LNKD- PUBMED:19681952, vol. 16, no. 7, 20 octobre 2009 (2009-10-20), pages 986-991, XP002612974, ISSN: 1469-0691
- JAIN SARJANA ET AL: "Rapid detection of extended-spectrum beta-lactamase-producing Gram-negative bacilli in blood cultures", JOURNAL OF ANTIMICROBIAL CHEMOTHERAPY, OXFORD UNIVERSITY PRESS, GB, vol. 60, no. 3, 1 septembre 2007 (2007-09-01), pages 652-654, XP002599185, ISSN: 0305-7453, DOI: DOI:10.1093/JAC/DKM256 cité dans la demande
- HIDEAKI HANAKI ET AL: "Substrate specificity of HMRZ-86 for Î-lactamases, including extended-spectrum Î-lactamases (ESBLs)", JOURNAL OF INFECTION AND CHEMOTHERAPY ; OFFICIAL JOURNAL OF THE JAPANESE SOCIETY OF CHEMOTHERAPY AND THE JAPANESE ASSOCIATION FORINFECTIOUS DISEASES, SPRINGER-VERLAG, TO, vol. 13, no. 6, 25 December 2007 (2007-12-25), pages 390-395, XP019567039, ISSN: 1437-7780
- T. LAURENT ET AL: 'Evaluation of the LACTA Test, a Novel Commercial Chromogenic Test for Rapid Detection of Ceftazidime-Nonsusceptible Pseudomonas aeruginosa Isolates' JOURNAL OF CLINICAL MICROBIOLOGY vol. 51, no. 6, 10 Avril 2013, pages 1951 - 1954, XP055165866 DOI: 10.1128/JCM.00524-13 ISSN: 0095-1137
- Scanvic ET AL: "Maldi-Tof Hémocultures poster 521: Utilisation du test chromogénique [beta]-LACTATM pour la détection rapide des entérobactéries résistantes aux céphalosporines de troisième génération directement sur les hémocultures", RICAI (Réunion interdisciplinaire de chimiothérapie anti-infectieuse), 21 November 2013 (2013-11-21), pages 1-4, XP055166142,
- DEFOURNY ET AL: 'P52. Evaluation du ßLACTA test sur hémocultures positives' 10E CONGRÈS NATIONAL DE LA SOCIÉTÉ FRANÇAISE DE MICROBIOLOGIE - LIVRE DES RÉSUMÉS 31 Mars 2014, pages 1 - 2, XP055166176
- Vandervinne ET AL: "P68. Evaluation du [beta]-LACTA test pour la détection de la résistance aux céphalosporines de troisième génération chez les bacilles Gram-négatifs directement dans les hémocultures", 10e Congrès National de la Société Française de Microbiologie - Livre des résumés, 31 March 2014 (2014-03-31), pages 1-2, XP055166181,
- ANONYMOUS: 'beta LACTA(TM) test: Détection rapide de la résistance aux céphalosporines de troisième génération chez les entérobactéries', [en ligne] 01 Février 2014, pages 1 - 9, XP055165884 Extrait de l'Internet: <URL:http://www.bio-rad.com/webroot/web/pdf /inserts/CDG/fr/68250_881150_FR.pdf> [extrait le 2015-01-29]

## Description

La présente invention concerne un procédé rapide de détection d'enzymes et de microorganismes.

Les procédés de détection de microorganismes dans un échantillon clinique comprennent généralement les étapes suivantes :
1) Traitement éventuel de l'échantillon de manière à favoriser la croissance bactérienne, la durée de cette étape variant en fonction de la nature de l'échantillon (sang, urines, pus, selles, liquide céphalo-rachidien, autres liquides de ponctions) et en fonction de la charge bactérienne;
2) Ensemencement d'un ou de plusieurs milieu(x) de croissance gélosé(s) sélectif(s) ou non ;
3) Incubation pendant 18 à 48 h à 37°C;
4) Observation des différents types de colonies obtenues (taille, aspect, couleur...)
5) Récupération d'un ou plusieurs isolat(s) de chaque type de colonies;
6) Identification proprement dite avec, si nécessaire, utilisation d'un automate ou d'une galerie d'identification;
7) Réalisation d'un antibiogramme en parallèle induisant un délai minimal de 16 h ou réalisation consécutive d'un antibiogramme induisant un délai supplémentaire de 18 h pour déterminer le profil du microorganisme vis-à-vis des antibiotiques et/ou des antifongiques.

Le diagnostic d'une infection par des microorganismes est donc généralement long, la durée dépendant notamment de la nature de l'échantillon à analyser. Ce délai d'attente est particulièrement problématique lorsque l'échantillon à analyser est un échantillon de sang. En effet, lorsque la présence de microorganismes est recherchée dans le sang, la première étape de traitement de l'échantillon en hémoculture, qui permet d'atteindre le seuil de détection du microorganisme, peut durer jusqu'à 7 jours. La durée de cette première étape de traitement ne peut être réduite car elle est dépendante des automates. Au moindre signe de positivité de la culture, une coloration de Gram est effectuée sur un échantillon prélevé par ponction aseptique de l'opercule à l'aide d'une seringue stérile. Cet examen direct va permettre de reconnaître la morphologie de l'agent bactérien présent dans le flacon ce qui peut orienter l'identification du germe. L'information est alors immédiatement transmise au clinicien car elle peut lui permettre d'instituer un traitement antibiotique non encore entrepris ou encore de rectifier une antibiothérapie probabiliste. Néanmoins aucune caractérisation précise du microorganisme en question et encore moins d'un profil de résistance particulier n'est possible à ce niveau. Il est donc particulièrement utile de pouvoir identifier ensuite rapidement les microorganismes présents dans l'échantillon biologique ainsi que leurs éventuels phénotypes de résistance afin de démarrer ou rectifier un traitement antibiotique.

Différents types de microorganismes peuvent être présents dans les hémocultures positives. Il est certain que les staphylocoques (non seulement *Staphylococcus aureus* mais aussi les staphylocoques à coagulase négative) sont régulièrement retrouvés. Les bacilles à Gram négatif, surtout les *Escherichia coli* (et plus généralement les entérobactéries), les bactéries anaérobies du côlon, les membres du groupe *Klebsiella*/*Enterobacter*, *Pseudomonas aeruginosa, Proteus* spp, et *Providencia* spp, ont été détectés par exemple après un trauma ou après une chirurgie réalisée à un endroit déjà contaminé du corps. La détection de salmonelles dans le sang d'individus ayant une salmonellose systémique n'est pas rare. De nombreux autres genres microbiens ont été retrouvés à partir d'une culture d'échantillons sanguins tels que les streptocoques, entérocoques, *Brucella, Pasteurella,* pneumocoques, *Neisseria*, *Listeria*, *Clostridium,* corynebactéries, *Bacteroides,* bactéries du groupe hacek mais aussi des levures et des parasites.

Différentes techniques sont utilisées pour identifier précisément ces microorganismes et leurs éventuels phénotypes de résistance, mais les résultats ne sont généralement obtenus qu'au bout d'une période relativement longue.

Des prélèvements d'échantillons sont ainsi étalés sur milieu au sang frais et milieu au sang cuit, auxquels peuvent être adjoints d'autres milieux en fonction des résultats des examens microscopiques.

Pour chaque flacon positif, on réalise :
- la détermination de la morphologie des colonies,
- les tests à l'oxydase et à la catalase,
- une identification biochimique et antigénique pour identifier le microorganisme,
- des tests de sensibilité, évaluation des Concentrations Minimales Inhibitrices (CMI).

Il a été montré qu'il était possible d'effectuer un antibiogramme directement à partir d'une hémoculture positive en réalisant un inoculum équivalent à 0,5 McF à partir du bouillon d'hémoculture. Une gélose Mueller-Hinton est inoculée par technique "d'inondation". La corrélation avec l'antibiogramme normalisé serait retrouvée dans 95% des cas. Le résultat apparaît alors au plus tôt 16 h après la mise en évidence de l'hémoculture positive (Antibiotics In Laboratory Madicine, Victor Lorian, M.D. Editor, 5^{ème} édition ; Doern et al., 1981, Antimicrob Agents Chemother. 20(5): 696-698. Antimicrobial Agents).

Il existe donc un besoin important de nouvelles méthodes plus rapides de détection de microorganismes, et/ou de leurs possibles résistances associées, dans un échantillon biologique, ces méthodes devant conserver une sensibilité et une spécificité satisfaisantes.

Les β-lactamines représentent la famille d'antibiotiques la plus importante du fait du grand nombre de molécules qui en font partie et du fait de leurs propriétés pharmacologiques et des spectres associés qui permettent de combattre la plupart des espèces bactériennes. Les β-lactamines sont à juste titre les antibiotiques les plus prescrits en médecine générale. Leur spectre d'activité est variable en fonction de leur classe (pénicillines ou céphalosporines) et parfois en fonction des molécules dans chaque classe. Ainsi, les pénicillines G et V sont plutôt actives sur les cocci à Gram positif et les bactéries anaérobies, tandis que le spectre des aminopénicillines s'étend à quelques bacilles à Gram négatif. L'adjonction d'un inhibiteur de β-lactamases permet en outre d'observer une activité sur certaines bactéries produisant ces β-lactamases. Les céphalosporines étendent encore le spectre d'activité vers les bacilles à Gram négatif mais sont un peu moins actives sur les cocci à Gram positif.

Parmi les β-lactamines, les céphalosporines représentent la catégorie d'antibiotiques la plus souvent prescrite. L'usage courant des céphalosporines a entraîné la diffusion de souches résistantes vis-à-vis de ces molécules. Ces souches parviennent notamment à survivre à la pression de sélection des céphalosporines de part une activité β-lactamase. La détection des résistances combinées vis-à-vis des céphalosporines de 1^{ère}, 2^{ème} et de 3^{ème} génération (C3G) chez les entérobactéries devient d'une importance thérapeutique majeure. En effet, la résistance des entérobactéries aux antibiotiques connaît une évolution mondiale préoccupante avec un impact croissant des β-lactamases à spectre étendu (BLSE) qui diffusent notamment dans le secteur communautaire. En 2002, moins de 1 % des souches d'entérobactéries avaient une BLSE. En 2006, elles représentaient 1 à 5 % des souches.

La résistance aux C3G, qu'elle soit due aux BLSE ou à d'autres β-lactamases (céphalosporinase ou carbapénémase) est donc en augmentation et est devenue significative, notamment pour *Escherichia coli* et *Klebsiella pneumoniae.* L'émergence de ces enzymes a récemment été rapportée chez des bacilles à Gram négatif non fermentants tels que *Acinetobacter baumannii, Pseudomonas aeruginosa* et *Stenotrophomonas maltophilia.* Il est donc de plus en plus important de pouvoir détecter ces BLSE.

La détection des BLSE chez les entérobactéries est simple à mettre en oeuvre dans la plupart des cas : elle est objectivée par une synergie entre le mélange [amoxicilline + acide clavulanique (AMC)] et une C3G (la ceftazidime est la plus sensible) ou l'aztréonam. Néanmoins, l'obtention d'un résultat demande beaucoup de temps. Le test de synergie est réalisé en disposant les disques d'AMC et de la C3G choisie (ou de l'aztréonam) à 30 mm de distance, centre à centre. La détection des BLSE est en revanche plus difficile chez les souches également hyperproductrices de céphalosporinase (AmpC) comme *Enterobacter.* Dans ce dernier cas, il est plus aisé de visualiser la synergie entre AMC et céfépime ou cefpirome. Enfin, chez certaines espèces telles que *Proteus mirabilis, Proteus vulgaris, Proteus penneri, Morganella morganii, Providencia stuartii* et *Providencia rettgeri,* les BLSE s'expriment faiblement et sont donc encore plus difficiles à détecter. Dans ces cas, le test de synergie est optimisé en plaçant les disques à une distance de 40-45 mm, au lieu de 30 mm, selon les recommandations du CA-SFM (Comité de l'antibiogramme de la Société française de microbiologie) en 2007. Des automates de bactériologie pour identification et antibiogramme sont de plus en plus utilisés (Mini-Api^{®} commercialisé par bioMérieux Clinical Diagnostics, Phoenix^{®} commercialisé par BD Diagnostics, MicroScan^{®} commercialisé par Siemens, Vitek^{®}, Vitek-2^{®} et Vitek Compact^{®} commercialisés par bioMérieux). D'une manière générale, ces automates détectent relativement bien les BLSE chez les souches qui n'hyperproduisent pas habituellement de céphalosporinases (*E. coli, K. pneumoniae, K. oxytoca*). Pour les autres espèces (*Enterobacter, Citrobacter, Serratia,* etc.), leur sensibilité et surtout leur spécificité sont un peu moins bonnes que celles des méthodes manuelles. Des tests complémentaires s'avèrent alors nécessaires.

La détection de souches résistantes aux C3G peut donc être aisée pour certaines résistances reliées à certaines espèces mais, d'une manière générale, aucune procédure ne possède 100 % de sensibilité ni ne permet d'obtenir un résultat rapide.

De nombreuses améliorations ont été proposées pour accélérer le processus de la détection et de l'identification des microorganismes présents en particulier dans les hémocultures. Tout d'abord, les améliorations des milieux de culture et des techniques de croissance ont réduit les délais de culture. La dernière génération d'automates peut même détecter une faible croissance bactérienne. Indépendamment des techniques classiques, quand la croissance est détectée par l'automate, il est également possible d'effectuer une identification directe des bactéries par biologie moléculaire (amplification et séquençage, FISH, puces à ADN ou sondes spécifiques,...). Néanmoins, la plupart de ces systèmes ne sont habituellement pas ouverts et permettent seulement la détection d'un seul ou d'un petit nombre de microorganismes spécifiques. De plus, ils peuvent ne pas fournir d'information au sujet de la susceptibilité ou de la résistance présumée envers un antibiotique. Par ailleurs, ces méthodes sont efficaces mais chères et/ou exigent des qualifications élevées des techniciens de laboratoire.

Des tentatives pour accélérer la détection plus spécifique des microorganismes résistants à une famille d'antibiotique ont été décrites. Ainsi, Weinbren et Borthwick (Weinbren et Borthwick, 2005, J. Antimicrob. Chemother. 55:131-132) ont décrit un procédé de détection non chromogénique de microorganismes produisant des BLSE, dans une hémoculture, dans lequel un échantillon de l'hémoculture est prélevé et étalé sur un milieu gélosé, des disques de cefpodoxime et cefpodoxime-clavulanate étant ensuite appliqués sur le milieu. Néanmoins, cette étape de culture sur milieu gélosé nécessite une attente supplémentaire minimale de 3,5 h à 6 h avant de pouvoir obtenir un premier résultat après la fin du traitement en hémoculture.

Navon-Venezia *et al.* (Navon-Venezia et al., 2005, J. Clin. Microb. 43:439-441) ont décrit un procédé de détection de bactéries produisant des BLSE à partir d'hémocultures positives consistant à prélever un échantillon du bouillon d'hémoculture et à l'ensemencer directement sur un milieu Mueller-Hinton contenant des disques cefpodoxime et cefpodoxime + clavulanate, sans passer par une étape d'isolement sur milieu gélosé. Aucun agent chromogène n'est mis en oeuvre dans ce procédé, qui nécessite une étape de culture de 16 h à 18 h conformément aux recommandations préconisées dans le référentiel américain CLSI/NCCLS (Clinical and Laboratory Standards Institute / National Committee on Clinical Laboratory Standards).

Chapin et Musgnug (2003) (Chapin et Musgnug, 2003, J. Clin Microb. 41:4751-4754) ont décrit un procédé direct de test de la sensibilité à des antimicrobiens à partir d'hémocultures positives, consistant à centrifuger 10 ml de l'hémoculture inoculée dans un tube séparateur de sérum contenant un gel. Les microorganismes restant à la surface du gel sont ensuite récupérés et remis en suspension afin d'inoculer des microplaques comprenant différentes dilutions d'antibiotiques spécifiques. Le résultat n'est toutefois obtenu que 18 à 24 h après l'inoculation.

Chen *et al.* (2008) (Chen et al., 2008, J. Microbiol. Immunol. Infect. 41:259-264) ont décrit un procédé d'identification de sensibilité de microorganismes à un groupe d'antibiotiques à partir d'hémocultures positives, consistant à prélever une hémoculture positive, éliminer les hématies par lyse et centrifugation, mettre en suspension le culot bactérien dans une solution saline et le tester en système Vitek-2^{®} ou AST (antimicrobial susceptibility testing). Il est néanmoins nécessaire d'attendre entre 2h30 et 16h15 après le chargement en système Vitek-2^{®} ou AST avant de pouvoir observer les premiers résultats.

L'équipe de Jain *et al.* (Jain et al., 2007, J. Antimicrob. Chemother. 60:652-654) a également décrit un procédé relativement rapide de détection de β-lactamases à spectre étendu (BLSE) par coloration à partir de surnageant de bouillon d'hémoculture après centrifugation. Du fait de problèmes d'hémolyse interférant avec le changement de couleur de l'agent chromogène, les auteurs ont proposé d'effectuer préalablement au test une sous-culture dans un milieu frais, allongeant ainsi la durée du procédé.

Ces techniques détectant les microorganismes résistants à la suite d'une étape de sous-culture restent donc relativement longues.

La demande internationale WO 2009/051838 décrit par ailleurs une méthode de détection de β-lactamases comprenant des étapes de mise en contact d'un échantillon biologique avec un substrat chromophore qui peut être le composé HMRZ-86, et différents inhibiteurs, permettant ainsi la discrimination des différentes β-lactamases, mais n'incluant pas d'étape de concentration des microorganismes présents dans l'échantillon et de resuspension des microorganismes dans une solution comprenant le composé HMRZ-86.

Les inventeurs ont mis au point un procédé de détection plus rapide, permettant de détecter des phénotypes de résistances précises telles que celles conférées aux bactéries à Gram négatif vis-à-vis des céphalosporines de 3^{ème} génération, directement à partir de l'échantillon clinique, ou après une étape de sous-culture, mais sans passer par une étape de sélection d'isolat sur milieu gélosé, et utilisant un substrat chromogène ou fluorogène.

La présente invention concerne un procédé de détection *in vitro* d'une enzyme d'un microorganisme résistant aux céphalosporines de 3^{ème} génération à partir d'un échantillon biologique, ladite enzyme à détecter étant sélectionnée dans le groupe constitué par les β-lactamases à spectre étendu, les céphalosporinases et les carbapénémases, ledit procédé comprenant les étapes consistant à :
a1) concentrer les microorganismes présents dans l'échantillon biologique, éventuellement après une étape a0) de culture des microorganismes;
b1) mettre en suspension les microorganismes concentrés à l'étape a1) dans une solution comprenant au moins un substrat chromogène susceptible de libérer un chromophore après hydrolyse par l'enzyme à détecter, ledit substrat chromogène étant le composé HMRZ-86 ((7R)-7-[2-(aminothiazol-4-yl)-(z)-2-(1-carboxy-1-methylethoxyimino)acetamido]-3-(2,4-dinitrostyryl)-3-cephem-4-carboxylic acid thrifluoroacetate, isomère E) ou un sel de celui-ci;
c1) détecter l'éventuelle libération du chromophore obtenue à l'étape b1);
   la libération du chromophore détectée à l'étape c1) étant indicative de la présence de l'enzyme à détecter et
ledit procédé comprenant en outre, lorsque l'échantillon biologique est un échantillon contenant du sang ou des hématies:
- une étape de lyse des hématies présentes dans l'échantillon biologique avant l'étape b1) de mise en suspension ou avant l'étape a1) de concentration, et/ou
- une étape a') de préparation de l'échantillon biologique avant l'étape a1) de concentration de microorganismes, l'étape a') comprenant :
   (i) l'agglutination des hématies, et
   (ii) la séparation des hématies agglutinées des microorganismes présents dans l'échantillon.

La présente demande décrit un procédé de détection *in vitro* d'une enzyme d'un microorganisme à partir d'un échantillon biologique comprenant les étapes consistant à :
a1) concentrer les microorganismes présents dans l'échantillon biologique, éventuellement après une étape a0) de culture des microorganismes;
b1) mettre en suspension les microorganismes concentrés à l'étape a1) dans une solution comprenant au moins un substrat chromogène ou fluorogène susceptible de libérer un chromophore ou un fluorophore après hydrolyse par l'enzyme à détecter;
c1) détecter l'éventuelle libération du chromophore ou du fluorophore obtenue à l'étape b1);
la libération du chromophore ou du fluorophore détectée à l'étape c1) étant indicative de la présence de l'enzyme à détecter.

La présente demande décrit également un procédé de préparation *in vitro* d'un échantillon d'hémoculture contenant des microorganismes, comprenant les étapes consistant à:
A) lyser ou agglutiner les hématies présentes dans l'échantillon d'hémoculture sans lyser les microorganismes présents dans l'échantillon d'hémoculture,
B) séparer les microorganismes présents dans l'échantillon d'hémoculture des hématies lysées ou agglutinées à l'étape A), et
C) éventuellement laver les microorganismes de l'échantillon d'hémoculture séparés à l'étape B).

La présente demande décrit par ailleurs un procédé de détection *in vitro* de microorganismes à partir d'un échantillon biologique mettant en oeuvre le procédé de détection d'enzyme ci-dessus.

### Description détaillée de l'invention

Dans le contexte de l'invention, un "échantillon biologique" fait référence à une substance d'origine biologique. De préférence, l'échantillon biologique est un échantillon d'un fluide biologique. Des exemples d'échantillons biologiques comprennent, mais ne sont pas limités à, le sang et ses composants, les urines, les selles, le liquide céphalo-rachidien ou autres liquides de ponction. De préférence, l'échantillon biologique selon l'invention est sélectionné dans le groupe constitué d'un échantillon de sang et d'un échantillon d'urine.

### Enzymes

Dans le contexte de l'invention, l'expression "enzyme d'un microorganisme" fait référence à une macromolécule de nature protéique exprimée par un microorganisme, et qui est caractérisée par son activité catalytique gouvernant des réactions biochimiques spécifiques au sein du microorganisme ou à l'extérieur du microorganisme lorsque cette enzyme est sécrétée par le microorganisme.

De préférence, l'enzyme décrite ici est sélectionnée dans le groupe constitué des glycosidases, des estérases, des phosphatases, des β-lactamases, en particulier les pénicillinases, les céphalosporinases, les carbénicillinases, les oxacillinases, les carbapénémases, les métallo-β-lactamases et les β-lactamases à spectre étendu. De manière davantage préférée, l'enzyme décrite ici est sélectionnée dans le groupe constitué des β-lactamases à spectre étendu, des céphalosporinases, des carbapénémases, des glycosidases et des estérases. L'enzyme selon l'invention est sélectionnée dans le groupe constitué par les β-lactamases à spectre étendu, les céphalosporinases et les carbapénémases. De préférence, l'enzyme est une enzyme capable d'hydrolyser les céphalosporines de 3^{ème} génération.

Par "glycosidase" ou "osidase", on entend ici une enzyme du groupe des hydrolases agissant sur la liaison glycosidique des oligosides et des glycosides. Comme il est bien connu de l'homme du métier, la spécificité d'une glycosidase est fonction de la nature du sucre lié par liaison glycosidique. Dans la nomenclature des enzymes, les glycosidases appartiennent à la classe 3 (correspondant aux hydrolases), sous-classe 2 (code EC3.2). Les glycosidases comprennent en particulier les glucosidases, les xylanases, les galactosidases, les lactases, les amylases, les chitinases, les fructosidases, les maltases, les neuraminidases, les invertases, les hyaluronidases et les lysozymes. Plus particulièrement, les glycosidases sont sélectionnées dans le groupe constitué de la N-acétyl-β-galactosaminidase, la N-acétyl-β-glucosaminidase, l'α-amylase, l'α-arabinofuranosidase, l'α-arabinosidase, la β-cellobiosidase, la β-chitobiosidase, l'α-fucosidase, la β-fucosidase, l'α-galactosidase, la β-galactosidase, l'α-glucosidase, la β-glucosidase, la β-glucuronidase, l'α-maltosidase, la β-maltosidase, l'α-mannosidase, la β-mannosidase et la β-xylosidase.

Par "estérase", on entend ici une enzyme du groupe des hydrolases clivant les esters en acide et alcool. Comme il est bien connu de l'homme du métier, les estérases diffèrent selon leur spécificité au substrat, leur structure protéique et leur fonction biologique. Dans la nomenclature des enzymes, les estérases appartiennent à la classe 3 (correspondant aux hydrolases), sous-classe 1 (code EC3.1). Les estérases comprennent en particulier les hydrolases de monoester triphosphorique, les sulfatases, les hydrolases de monoester diphosphorique, les hydrolases de triester phosphorique, les exodésoxyribonucléases, les exoribonucléases, les exonucléases, les désoxyribonucléases, les ribonucléases, les endodésoxyribonucléases et les endoribonucléases.

Par "phosphatase", on entend ici une enzyme éliminant un groupe phosphate d'une molécule simple ou d'une macromolécule biologique, en hydrolysant les monoesters d'acide phosphorique en un ion phosphate et une molécule avec un groupement hydroxyle libre. Dans la nomenclature des enzymes, les phosphatases appartiennent à la classe 3 (correspondant aux hydrolases), sous-classe 1.3 (code EC3.1.3). Comme il est bien connu de l'homme du métier, les phosphatases diffèrent selon leur spécificité au substrat. Les phosphatases comprennent en particulier les tyrosine phosphatases, les sérine/thréonine phosphatases, les phosphatases à double spécificité, les histidine phosphatases et les lipide phosphatases.

Par "β-lactamase", on entend ici une enzyme qui hydrolyse les β-lactamines avec ouverture du noyau β-lactame et production de dérivés inactifs. Les β-lactamases sont généralement sécrétées dans le milieu extérieur chez les bactéries à Gram positif, et dans l'espace périplasmique chez les bactéries à Gram négatif. Elles regroupent entre autres les céphalosporinases, les β-lactamases à spectre étendu et les carbapénémases. Les β-lactamases sont classées selon deux classifications principales:
- la classification de Bush, élaborée en 1989 et réactualisée en 1995 puis en 2009, classe les β-lactamases en fonction de leur substrat préférentiel parmi la pénicilline, l'oxacilline, la carbénicilline, la céfaloridine, le céfotaxime et l'imipénème, et en fonction de leur sensibilité à l'acide clavulanique, un inhibiteur des β-lactamases;
- la classification de Ambler, proposée en 1980, est basée sur la séquence protéique des β-lactamases. Elle se compose de quatre classes d'enzymes : A, B, C et D. Les β-lactamases des classes A, C et D comportent une sérine au niveau du site actif, qui forme une liaison covalente transitoire avec la β-lactamine, entraînant l'ouverture du noyau β-lactame. Ces β-lactamases sont aussi appelées sérine β-lactamases. La classe B regroupe quant à elle des métallo-enzymes nécessitant un ion zinc pour hydrolyser le noyau β-lactame.

Les β-lactamases sont nombreuses et diversifiées, en fonction de leurs propriétés. Elles sont responsables de la résistance aux β-lactamines comme les pénicillines, les céphalosporines, les monobactames, les céphamycines, et les carbapénèmes. Elles inactivent ces antibiotiques par hydrolyse du noyau β-lactame. Parmi les β-lactamases, la β-lactamase TEM-1 est très largement répandue dans les différentes espèces bactériennes. Elle hydrolyse très efficacement les pénicillines, mais pas les céphalosporines de 3^{ème} génération, et elle est sensible à l'inhibition par l'acide clavulanique. Les bactéries produisant des β-lactamases de cette catégorie sont donc facilement traitées par des céphalosporines de 3^{ème} génération (C3G). En revanche, les BLSE, certaines céphalosporinases et carbapénémases, lorsqu'elles sont produites par des bactéries, les rendent résistantes aux céphalosporines de 3^{ème} génération (C3GR).

Les séquences en acides aminés des β-lactamases sont très proches les unes des autres au sein d'une même famille. Il a ainsi suffi de la substitution d'un petit nombre d'acides aminés pour permettre aux β-lactamases de type TEM-1, initialement inactives, d'hydrolyser les céphalosporines β-lactamases de type BLSE par exemple), ou de devenir résistantes aux inhibiteurs.

Le **tableau 1** suivant décrit les propriétés des différents types de β-lactamases, en particulier des céphalosporinases, des β-lactamases à spectre étendu et des carbapénémases.

**Tableau 1: Tableau synthétique et non exhaustif décrivant les propriétés des β-lactamases majeures conférant (C3GR) ou non la résistance aux C3G.**

| | Exemples | Inhibé par le clavulanate | Classe moléculaire (Ambler) | C3GR |
|---|---|---|---|---|
| β-lactamase à spectre large (pénicillinases) | TEM-1, TEM-2, SHV-1 | +++ | A | Non |
| | Famille OXA (OXA-1) | + | D | Non |
| β-lactamase à spectre étendu (BLSE) | Familles TEM, SHV, CTX-M | ++++ | A | Oui |
| | Famille OXA (OXA-11,-14,-15,-16,-17) | + | D | Oui |
| céphalosporinases ou AmpC | Chromosomiques | 0 | C | Non |
| | Hyperproduites Plasmidiques : ACC-1, famille CMY, DHA-1 | 0 | C | Oui |
| carbapénémases | Familles IMP, VIM (métallo-β-lactamases) | 0 | B | Oui |
| | Famille KPC | +++ | A | Oui |
| | Famille OXA (OXA 23-27, OXA-40, OXA-48) | + | D | Oui |

### Microorganismes

Par "microorganisme", on entend ici un organisme vivant présentant une structure cellulaire eucaryote ou procaryote, ou qui est acaryote, et qui est caractérisé par l'unicellularité, une taille microscopique ou ultramicroscopique et un potentiel métabolique et de reproduction. Les microorganismes selon l'invention comprennent en particulier les bactéries, notamment les bactéries à Gram négatif et les bactéries à Gram positif, et les champignons, notamment les levures (par exemple du genre *Candida).* Plus spécifiquement, il peut s'agir d'une bactérie sélectionnée dans le groupe constitué:
- des Enterobacteriaceae tels que les souches du genre *Klebsiella,* en particulier *Klebsiella pneumoniae* et *Klebsiella oxytoca*; les souches du genre *Escherichia*, en particulier *Escherichia coli;* les souches du genre *Enterobacter,* en particulier *Enterobacter cloacae, Enterobacter asburiae* et *Enterobacter aerogenes;* les souches du genre *Citrobacter,* en particulier *Citrobacter freundii* et *Citrobacter koseri*; les souches du genre *Proteus,* en particulier *Proteus mirabilis, Proteus vulgaris* et *Proteus rettgeri*; les souches du genre *Serratia,* en particulier *Serratia marcescens*; les souches du genre *Salmonella*, les souches du genre *Providencia,* les souches du genre *Shigella* et les souches du genre *Kluyvera, Morganella morganii* et *Hafnia alvei*;
- des bacilles à Gram négatif non fermentants tels que les souches du genre *Acinetobacter,* en particulier *Acinetobacter baumannii*; les souches du genre *Pseudomonas*, en particulier *Pseudomonas aeruginosa,* les souches du genre *Stenotrophomonas,* et les souches du genre *Burkholderia*;
- des bactéries à Gram négatif anaérobies telles que *Bacteroides fragilis*;
- des coccobacilles ou cocci à Gram négatif tels que *Haemophilus influenzae*, *Bordetella* et *Neisseria spp ;*
- des bacilles ou cocci à Gram positif tels que les souches du genre *Lactobacillus;* les souches du genre *Enterococcus;* les souches du genre *Streptococcus;* les souches du genre *Staphylococcus,* en particulier les souches de *Staphylococcus aureus* résistantes ou non à la méticilline; les souches du genre *Listeria*; et les souches du genre *Clostridium.*

De manière préférée, les microorganismes décrits ici sont sélectionnés dans le groupe constitué des microorganismes portant ou pouvant acquérir une résistance aux céphalosporines de 3^{ème} génération. En particulier, les microorganismes selon l'invention sont sélectionnés dans le groupe constitué de *Klebsiella pneumoniae, Escherichia coli*, *Proteus mirabilis, Proteus rettgeri, Proteus vulgaris, Enterobacter cloacae, Enterobacter aerogenes, Klebsiella oxytoca*, *Salmonella spp., Pseudomonas aeruginosa, Acinetobacter baumannii*, *Stenotrophomonas maltophilia*, *Serratia marcescens, Citrobacter freundii*, *Citrobacter koseri*, *Morganella morganii*, et *Providencia sp.*

Les microorganismes selon l'invention sont des microorganismes résistants aux céphalosporines de 3^{ème} génération.

Quand le microorganisme selon l'invention est résistant aux céphalosporines de 3^{ème} génération, et est donc en général également résistant aux céphalosporines de 1^{ère} et 2^{ème} générations et aux pénicillines, l'enzyme telle que définie ci-dessus est sélectionnée dans le groupe constitué par les β-lactamases à spectre étendu, les céphalosporinases et les carbapénémases.

De préférence, quand le microorganisme selon l'invention est résistant aux céphalosporines de 3^{ème} génération, l'enzyme est une enzyme capable d'hydrolyser les céphalosporines de 3^{ème} génération. De manière préférée entre toutes, quand le microorganisme selon l'invention est résistant aux céphalosporines de 3^{ème} génération, l'enzyme selon l'invention est une β-lactamase à spectre étendu.

Dans le contexte de l'invention, l'expression "microorganisme résistant aux céphalosporines de 3^{ème} génération" fait référence à des microorganismes qui, de part l'activité spécifique de leur(s) β-lactamase(s), continuent de se multiplier et/ou qui ne meurent pas quand ils sont cultivés en présence d'une concentration classiquement inhibitrice de céphalosporines de 3^{ème} génération.

De préférence, les "microorganismes résistants aux céphalosporines de 3^{ème} génération" selon la présente invention sont sélectionnés dans le groupe constitué de *Klebsiella pneumoniae, Escherichia coli, Proteus mirabilis, Enterobacter cloacae, Enterobacter aerogenes, Klebsiella oxytoca, Salmonella spp., Pseudomonas aeruginosa*, *Acinetobacter baumannii*, *Stenotrophomonas maltophilia*, *Proteus rettgeri*, *Proteus vulgaris, Serratia marcescens, Citrobacter freundii*, *Citrobacter koseri*, *Morganella morganii* et *Providentia sp..*

### Substrat chromogène ou fluorogène

Dans le contexte de l'invention, l'expression "substrat chromogène" fait référence à une molécule pouvant être clivée ou modifiée par une enzyme et qui comprend ou est couplée à un chromophore.

Par "chromophore", on entend ici un groupement d'atomes au sein d'une molécule qui est responsable des propriétés d'absorption et/ou d'émission de lumière dans le domaine de l'ultraviolet, du visible ou de l'infrarouge de cette molécule. Ces propriétés résultent d'une capacité à absorber l'énergie de photons dans une gamme du spectre visible tandis que les autres longueurs d'onde sont transmises ou diffusées.

Le substrat chromogène décrit ici peut être coloré ou incolore. Ce substrat chromogène libère son chromophore sous l'action d'une enzyme spécifique.

Comme décrit ici, l'expression "substrat fluorogène" fait référence à une molécule pouvant être clivée ou modifiée par une enzyme et qui comprend ou est couplée à un fluorophore. Ce substrat fluorogène libère son fluorophore sous l'action d'une enzyme spécifique.

Par "fluorophore", on entend ici un groupement d'atomes au sein d'une molécule qui est responsable de la capacité de cette molécule à émettre de la lumière de fluorescence après excitation. Ce sont généralement des substances composées de plusieurs noyaux aromatiques conjugués ou encore des molécules planes et cycliques qui possèdent une ou plusieurs liaisons π.

Des chromophores ou fluorophores sont bien connus de l'homme du métier et couramment utilisés en laboratoire depuis de nombreuses années (voir par exemple Vinazzer (1975) Haemostasis 4:101-9, Manafi (2000) Int. J. Food Microbiol. 60:205-218, Orenga et al. (2009) J. Microbiol. Methods 79:139-155).

Le chromophore peut par exemple correspondre à un dérivé de l'indoxyle (par exemple le 3-indolyl-R, le 5-bromo-3-indolyl-R, le 5-bromo-4-chloro-3-indoxyl, le 5-bromo-6-chloro-3-indoxyl ou le 6-chloro-3-indoxyl), à un dérivé de l'indol (par exemple le 7-amido-5-bromoindole), au nitrophénol ou l'un de ses dérivés (par exemple le paranitrophénol, l'orthonitrophénol ou l'orthofluorophénol), au chlorophénol ou l'un de ses dérivés (par exemple le 4-amino-2,6-dichlorophénol), au naphthol ou l'un de ses dérivés (par exemple le 1-naphthol ou le 2-naphtylamide), au 5-(4-hydroxy-3-methoxyphenylmethylene)-2-thioxothiazolidin-4-one-3-ethanoate, la 3,4-cyclohexenoesculetine, la 2-alizarine ou la 7-amido-1-pentyl-phenoxazin-3-one.

Le fluorophore peut par exemple correspondre à la coumarine ou l'un de ses dérivés (par exemple l'hydroxycoumarine, l'aminocoumarine, la 7-amido-4-méthylcoumarine, la méthoxycoumarine, l'acide 7-nitrocoumarine-3-carboxylique), la phycoérythrine, la fluorescéine ou l'un de ses dérivés (par exemple la 5-dodecanoylaminofluorescéine), la 4-méthyl-umbelliféryle, la résofurine, la rhodamine, l'allophycocyanine, la 2-(5'-chloro-2'-hydroxyphényl)-6-chloro-4-(3H)-quinazolinone.

Comme décrit ici, l'expression "substrat chromogène ou fluorogène susceptible de libérer un chromophore ou fluorophore après hydrolyse par l'enzyme à détecter" fait référence à un substrat chromogène ou fluorogène tel que défini ci-dessus qui, lorsqu'il est mis en contact avec l'enzyme dont il est spécifique, libère le chromophore ou fluorophore qu'il comprend ou avec lequel il est couplé.

La libération du chromophore ou fluorophore peut être due directement ou indirectement à l'hydrolyse du substrat par l'enzyme à détecter. Ainsi, l'enzyme à détecter peut hydrolyser la liaison couplant le substrat au chromophore ou fluorophore, libérant ainsi le chromophore ou fluorophore du substrat. Cette action directe de l'enzyme est typiquement observée avec les substrats couplés à un chromophore ou fluorophore. L'enzyme à détecter peut également hydrolyser un domaine du substrat n'impliquant pas le chromophore ou fluorophore.

Par conséquent, la détection de la libération du chromophore ou du fluorophore du substrat chromogène ou fluorogène décrit ici indique que ce substrat a été hydrolysé par une enzyme spécifique.

De préférence, la libération du chromophore ou du fluorophore entraîne un changement de couleur du substrat chromogène ou d'émission de fluorescence du substrat fluorogène. Par conséquent, la détection de la libération du chromophore ou du fluorophore peut en particulier être mise en oeuvre en observant le changement de couleur du substrat chromogène ou d'émission de fluorescence du substrat fluorogène.

De préférence, le substrat chromogène ou fluorogène décrit ici est un substrat ou un dérivé d'un substrat d'une enzyme telle que définie ci-dessus. En particulier, un substrat chromogène ou fluorogène décrit ici est un substrat ou un dérivé d'un substrat d'une enzyme sélectionnée dans le groupe constitué des glycosidases, des estérases, des phosphatases, des β-lactamases, en particulier les pénicillinases, les céphalosporinases, les carbénicillinases, les oxacillinases, les carbapénémases dont les métallo-β-lactamases, et les β-lactamases à spectre étendu. Plus particulièrement, un substrat chromogène ou fluorogène décrit ici est un substrat ou un dérivé d'un substrat d'une enzyme sélectionnée dans le groupe constitué des céphalosporinases, des carbapénémases dont les métallo-β-lactamases, et des β-lactamases à spectre étendu.

Par "dérivé d'un substrat d'une enzyme", on entend ici un composé issu d'un substrat d'une enzyme, capable d'être hydrolysé par les mêmes enzymes que le substrat à partir duquel il a été produit. De préférence, un dérivé d'un substrat d'une enzyme décrit ici est un substrat modifié de manière à contenir ou à être couplé à un chromophore ou un fluorophore.

Des substrats spécifiques d'enzymes sont bien connus de l'homme du métier. Des exemples de substrats de glycosidases comprennent ainsi les glycosides, les acides uroniques, les sucres aminés et les sucres acétylés. Des exemples de substrats d'estérases comprennent en particulier les butyrates, les palmitates, les stéarates, les oléates, les laurates et les caprylates. Des exemples de substrats de phosphatases comprennent en particulier les esters de phosphate d'alcool d'alkyle, les esters de phosphate d'aryle, les esters de phosphate d'arylalkyle, les phosphates d'énol, les phosphates d'aryle, les phosphates de diaryle, le pyrophosphate inorganique, le pyrophosphate organique, les phosphamides et les thioesters. Des exemples de substrats de pénicillinases comprennent ainsi les pénicillines et la nitrocéfine. Les substrats de céphalosporinases comprennent par exemple la nitrocéfine, les pénicillines, les céphalosporines de 1^{ère} génération et certaines céphalosporines de 2^{ème} génération et de 3^{ème} génération (telles que les céphalosporines de 3^{ème} génération définies ci-dessous). Des exemples de substrats de carbénicillinases incluent la nitrocéfine, les carbénicillines, les pénicillines et les cloxacillines. Des exemples de substrats de oxacillinases incluent la nitrocéfine, les cloxacillines, les pénicillines et les carbénicillines. Des exemples de substrats de carbapénémases incluent les pénicillines, les céphalosporines, les carbapénèmes et la nitrocéfine. Des exemples de substrats de métallo-β-lactamases comprennent en particulier la nitrocéfine, les pénicillines, les céphalosporines et les carbapénèmes. Des exemples de substrats de β-lactamases à spectre étendu incluent la nitrocéfine, les pénicillines et les céphalosporines, en particulier les céphalosporines de 3^{ème} génération telles que définies ci-dessous.

Dans un mode de réalisation préféré, lorsque l'enzyme à détecter est sélectionnée dans le groupe constitué par les β-lactamases à spectre étendu, les céphalosporinases et les carbapénémases, le substrat chromogène ou fluorogène comprend un noyau β-lactame.

Par "noyau β-lactame", on entend ici une structure cyclique hétéroatomique, consistant en trois atomes de carbone et un d'azote. De préférence, le noyau β-lactame du substrat chromogène ou fluorogène décrit ici est hydrolysé par l'enzyme à détecter.

De manière davantage préférée, lorsque l'enzyme à détecter est sélectionnée dans le groupe constitué par les β-lactamases à spectre étendu, les céphalosporinases et les carbapénémases, le substrat chromogène ou fluorogène décrit ici est un dérivé de β-lactamine.

Par "β-lactamine", on entend ici un antibiotique contenant un noyau β-lactame dans sa structure moléculaire. Comme il est bien connu de l'homme du métier, les β-lactamines englobent par exemple les dérivés de la pénicilline, les céphalosporines, les monobactames et les carbapénèmes.

De manière encore préférée, lorsque l'enzyme à détecter est sélectionnée dans le groupe constitué par les β-lactamases à spectre étendu, les céphalosporinases et les carbapénémases, le substrat chromogène ou fluorogène décrit ici est un dérivé de céphalosporine.

Par "dérivé de céphalosporine", on entend ici une molécule composée, d'une part, d'un groupement issu d'une céphalosporine, ou partie de céphalosporine, capable d'être hydrolysé par une β-lactamase, et, d'autre part, d'un groupement chromophore ou fluorophore. De préférence, le dérivé de céphalosporine décrit ici est une céphalosporine modifiée de manière à contenir ou à être couplée à un chromophore.

Les céphalosporines sont habituellement classées en céphalosporines de première, de deuxième, de troisième génération ou de quatrième génération sur la base de leur spectre d'activité et de leur plus ou moins grande résistance ou stabilité vis-à-vis des β-lactamases. Cette classification est bien connue de l'homme du métier (voir par exemple Thompson (1987) Mayo Clin Proc. 62:821-34; Gustaferro et Steckelberg (1991) Mayo Clin Proc. 66:1064-73; et Barber et al. (2004) Adv Biochem Eng Biotechnol. 88:179-215). Des exemples de céphalosporines de 1^{ère} génération incluent en particulier la céfazoline, la céfalotine, la céfapirine, la céfaloridine, la céfalexine Keforal^{®}, la céfradine Zeefra^{®} et la céfradoxil Oracéfal^{®}. Des exemples de céphalosporines de 2^{ème} génération incluent entre autres la céfamandole, la céfuroxime Zinnat^{®}, la céfonicide, la céforanide, la céfatrizine, le céfotiam, le cefprozil, le loracarbef, le céfotétan, la céfotixine et le céfaclor Alfatil^{®}. Des exemples de céphalosporines de 4^{ème} génération incluent en particulier la céfépime et la cefpirome.

De manière préférée entre toutes, le substrat chromogène ou fluorogène décrit ici est un dérivé de céphalosporine de 3^{ème} génération.

Par "dérivé de céphalosporine de 3^{ème} génération", on entend ici un dérivé de céphalosporine tel que défini ci-dessus capable d'être hydrolysé par les mêmes enzymes que les céphalosporines de 3^{ème} génération. De préférence, un dérivé de céphalosporine de 3^{ème} génération décrit ici est une céphalosporine de 3^{ème} génération, ou une partie d'une céphalosporine de 3^{ème} génération, modifiée de manière à contenir ou à être couplée à un chromophore ou un fluorophore. De manière davantage préférée, le dérivé de céphalosporine de 3^{ème} génération décrit ici est une céphalosporine de 3^{ème} génération, ou une partie d'une céphalosporine de 3^{ème} génération, modifiée de manière à contenir ou à être couplée à un chromophore.

Les céphalosporines de 3^{ème} génération incluent notamment les composés dont les dénominations anglaises sont les suivantes : Cefcapene (Référence CAS: 135889-00-8), Cefcapene Pivoxil (Référence CAS: 105889-45-0), Cefcapene Pivoxil Hydrochloride (Référence CAS: 147816-23-7), Cefcapene Pivoxil Hydrochloride Monohydrate (Référence CAS: 147816-24-8), Cefdaloxime (Référence CAS: 80195-36-4), Cefdaloxime Pivoxil, Cefdinir (Référence CAS: 91832-40-5), Cefditoren (Référence CAS : 104146-53-4), Cefditoren Sodium (Référence CAS: 104146-53-4), Cefditoren Pivoxil (Référence CAS: 117467-28-4), Cefetamet Pivoxil Hydrochloride, Cefetamet Pivoxyl (Référence CAS: 65243-33-6), Cefetamet (Référence CAS: 65052-63-3), Cefixime (Référence CAS: 79350-37-1), Cefixime Trihydrate, Cefmenoxime Hydrochloride (Référence CAS: 75738-58-8), Cefmenoxime (Référence CAS: 65085-01-0), Cefodizime Sodium (Référence CAS: 86329-79-5), Cefodizime (Référence CAS: 69739-16-8), Cefoperazone Sodium (Référence CAS: 62893-20-3), Cefoperazone A, Cefoperazone (Référence CAS: 62893-19-0), Cefotaxime Sodium (Référence CAS: 64485-93-4), Cefotaxime S-oxide, Cefotaxime (Référence CAS: 63527-52-6), Benzathine Cefotaxime, Desacetylcefotaxime, Cefpimizole Sodium, Cefpimizole, Cefpiramide Sodium (Référence CAS: 74849-93-7), Cefpiramide (Référence CAS: 70797-11-4), Cefpodoxime Proxetil (Référence CAS: 87239-81-4), Cefpodoxime (Référence CAS: 80210-62-4), Cefpodoxime Hydrate, Cefsulodin Sodium, Cefsulodin, Ceftazidime Pentahydrate (Référence CAS: 78439-06-2), Ceftazidime (Référence CAS: 72558-82-8), Cefteram, Cefteram Pivaloyloxymethyl Ester, Ceftibuten, Trans-ceftibuten, Ceftiofur, Ceftiofur Sodium (Référence CAS: 80370-57-6), Ceftiofur Hydrochloride, Ceftiofur Sodium, Desfuroylceftiofur, Ceftiolene, Ceftizoxime Alapivoxil, Ceftizoxime Sodium, Ceftizoxime, Ceftriaxone Disodium (Référence CAS: 74578-69-1), Ceftriaxone Sodium, Ceftriaxone (Référence CAS: 73384-59-5) et les sels de ces composés. La structure et la dénomination IUPAC de ces composés peut par exemple être trouvée sur le site chemicalland21.com.

Comme décrit ici, la céphalosporine de 3^{ème} génération peut être choisie parmi le ceftriaxone (Rhocéphine^{®}), le céfotaxime (Claforan^{®}), le ceftazidime (Fortum^{®}), le céfixime (Oroken^{®}), le cefpodoxime proxetil (Orelox^{®}), le céfotiam ou le céfotiam hexetil (Takétiam^{®}), le Cefpirome (Cefrom^{®}), le Céfépime (Axépim^{®}), le Cefsulodine (Pyocéfal^{®}), le céfatamet, le ceftizoxime, le céfopérazone, le cefsulodin, le ceftibuten et les sels de ces composés.

Des exemples de substrats chromogènes spécifiques de β-lactamases sont bien connus de l'homme du métier et incluent en particulier la nitrocéfine (acide (3-[2,4-dinitrostyryl]-7-(2-thienylacetamido)3-cephem-4-carboxylique), le PADAC® (Pyridinium-2-azo-*p*-dimethylaniline chromophore), le CENTA™, le HMRZ-86 (trifluoroacétate d'acide (7R)-7[2-aminothiazol-4-yl]-(z)-2-(1-carboxy-1-methylethoxyimino)acetamido)-3-(2,4-dinitrostyryl)-3-cephem-4-carboxylique, isomère E) et la cefesone ou S1 (3-(2,4-dinitrostyryl)-(6*R*,7*R*)-7-phenylacetamido-ceph-3-em-4-carboxylate).

Des exemples de substrats fluorogènes spécifiques de β-lactamases sont bien connus de l'homme du métier et incluent en particulier la Fluorocilline Green 495/525 et la Fluorocilline Green 345/350 Live Blazer™-FRET B/G.

Comme décrit ici, le dérivé de céphalosporine de 3^{ème} génération contenant ou étant couplé à un chromophore peut être l'un des composés décrits dans la demande de brevet européen n° 1325923. Ces composés sont représentés par la formule (I) : 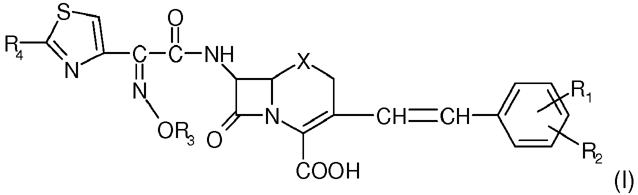 où :
- R₁ et R₂ peuvent être identiques ou différents et représentent chacun un atome d'hydrogène ou un groupement nitro ou cyano ;
- R₃ représente un groupement alkyle en C₁-C₆, éventuellement substitué par un groupement carboxyle;
- R₄ représente un atome d'hydrogène ou un groupement amino ;
- X représente -S- ou -SO- ; et
- R₁ et R₂ ne peuvent pas simultanément représenter un atome d'hydrogène.

Ainsi, le dérivé de céphalosporine de 3^{ème} génération contenant ou étant couplé à un chromophore peut par exemple être le composé:
- acide 7-[2-(2-aminothiazol-4-yl)-2-(1-carboxy-1-methylethoxy-imino)acetamido]-3-(2,4-dinitrostyryl)-3-cephem-4-carboxylique,
- acide 7-[2-(2-aminothiazol-4-yl)-2-(1-carboxy-1-methylethoxy-imino)acetamido]-3-(2,6-dinitrostyryl)-3-cephem-4-carboxylique,
- acide 7-[2-(2-aminothiazol-4-yl)-2-(1-carboxy-1-methylethoxy-imino)acetamido]-3-(4-nitrostyryl)-3-cephem-4-carboxylique,
- acide 7-[2-(2-aminothiazol-4-yl)-2-(1-carboxy-1-methylethoxy-imino)acetamido]-3-(2,4-dicyanostyryl)-3-cephem-4-carboxylique,
- acide 7-[2-(2-aminothiazol-4-yl)-2-(1-carboxy-1-methylethoxy-imino)acetamido]-3-(4-cyanostyryl)-3-cephem-4-carboxylique,
- acide 7-[2-(2-aminothiazol-4-yl)-2-(1-carboxy-1-methylethoxy-imino)acetamido]-3-(2-cyanostyryl)-3-cephem-4-carboxylique,
- acide 7-[2-(1-carboxy-1-methylethoxyimino)-2-(thiazol-4-yl)acetamido]-3-(2,4-dinitrostyryl)-3-cephem-4-carboxylique,
- acide 1-oxide-7-[2-(2-aminothiazol-4-yl)-2-(1-carboxy-1-methylethoxy-imino)acetamido]-3-(2,4-dinitrostyryl)-3-cephem-4-carboxylique,
- acide 7-[2-(2-aminothiazol-4-yl)-2-methoxyiminoacetamido]-3-(4-nitrostyryl)-3-cephem-4-carboxylique,
- acide 7-[2-(2-aminothiazol-4-yl)-2-methoxyiminoacetamido]-3-(2,4-dicyanostyryl)-3-cephem-4-carboxylique,
- acide 7-[2-(2-aminothiazol-4-yl)-2-methoxyiminoacetamido]-3-(2,6-dicyanostyryl)-3-cephem-4-carboxylique,
- acide 7-[2-(2-aminothiazol-4-yl)-2-methoxyiminoacetamido]-3-(2-cyanostyryl)-3-cephem-4-carboxylique,
- acide 7-[2-(2-aminothiazol-4-yl)-2-carboxymethoxyimino-acetamido]-3-(2,4-dinitrostyryl)-3-cephem-4-carboxylique,
- acide 7-[2-(2-aminothiazol-4-yl)-2-carboxymethoxyimino-acetamido]-3-(2,6-dinitrostyryl)-3-cephem-4-carboxylique,
- acide 7-[2-(2-aminothiazol-4-yl)-2-carboxymethoxyimino-acetamido]-3-(4-nitrostyryl)-3-cephem-4-carboxylique,
- acide 7-[2-(2-aminothiazol-4-yl)-2-carboxymethoxyimino-acetamido]-3-(2-nitrostyryl)-3-cephem-4-carboxylique,
- acide 7-[2-(2-aminothiazol-4-yl)-2-carboxymethoxyimino-acetamido]-3-(2,4-dicyanostyryl)-3-cephem-4-carboxylique,
- acide 7-[2-(2-aminothiazol-4-yl)-2-carboxymethoxyimino-acetamido]-3-(4-cyanostyryl)-3-cephem-4-carboxylique,
- acide 7-[2-(2-aminothiazol-4-yl)-2-carboxymethoxyimino-acetamido]-3-(2-cyanostyryl)-3-cephem-4-carboxylique,
- acide [2-carboxymethoxyimino-2-(thiazol-4-yl)acetamido]-3-(2,4-dinitrostyryl)-3-cephem-4-carboxylique,
- acide 1-oxide-7-[2-(2-aminothiazol-4-yl)-2-carboxymethoxyimino-acetamido]-3-(2,4-dinitrostyryl)-3-cephem-4-carboxylique,
ou un sel de ceux-ci,
tels que décrits dans la demande de brevet européen n° 1325923.

Dans le cadre de l'invention, le substrat chromogène est le composé HMRZ-86 ((7R)-7-[2-(aminothiazol-4-yl)-(z)-2-(1-carboxy-1-methylethoxyimino)acetamido]-3-(2,4-dinitrostyryl)-3-cephem-4-carboxylic acid thrifluoroacetate, isomère E) (Hanaki et al. (2004) Journal of Antimicrobial Chemotherapy 53:888-889). Ce composé est représenté par la formule (II) suivante :

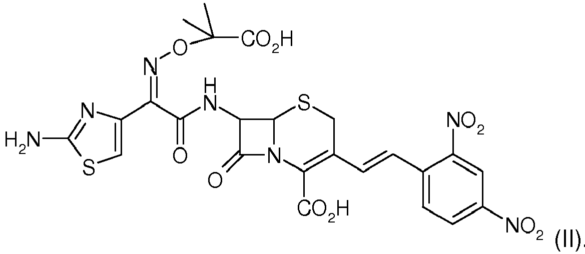

### Etape de culture

Dans certains modes de réalisation de l'invention, le nombre de microorganismes présents dans l'échantillon biologique est insuffisant pour observer la libération du chromophore ou du fluorophore du substrat chromogène ou fluorogène selon l'invention. Par conséquent, il peut être nécessaire de cultiver l'échantillon biologique de manière à permettre la croissance des microorganismes, avant de concentrer les microorganismes éventuellement présents et obtenir un nombre de microorganismes suffisant pour observer la libération du chromophore ou du fluorophore du substrat chromogène ou fluorogène.

Aussi, dans un mode préféré de l'invention, le procédé selon l'invention comprend une étape préalable a0) de culture des microorganismes. Cette étape préalable a0) de culture des microorganismes se fait dans des conditions appropriées pour permettre la croissance des microorganismes.

Par "conditions appropriées pour permettre la croissance des microorganismes", on entend ici des conditions de température, d'oxygénation, d'agitation appropriées, un milieu approprié, et une période appropriée pour que les microorganismes présents dans l'échantillon biologique puissent se multiplier. Les conditions appropriées pour permettre la croissance des microorganismes dépendent des microorganismes à détecter et sont bien connues de l'homme du métier. Typiquement, l'étape de culture a lieu dans un milieu de culture non sélectif, de préférence un milieu de culture liquide, tel que du milieu trypto - caséine - soja (TCS), à 37°C, pendant une période permettant d'obtenir une concentration finale permettant la réalisation du test.

De préférence, lorsque l'échantillon biologique est un échantillon de sang, l'étape a0) de culture est une étape d'hémoculture.

Par "hémoculture", on entend ici la culture d'un échantillon de sang circulant. Les conditions de cultures en hémoculture sont bien connues de l'homme du métier et sont, par exemple, décrites dans "Hémocultures, Garnier F. et Denis F., In : Bactériologie médicale : techniques usuelles (2007) Ed. Masson 11:107-116". Typiquement, l'échantillon de sang est mis en culture dans un milieu liquide non sélectif tel que du milieu coeur-cervelle, du milieu trypticase soja ou du bouillon de type milieu de Wilkins Chalgren, supplémenté avec des nutriments et des facteurs de croissance (par exemple des vitamines, hémine, hydrates de carbone, cystéine, etc...), comprenant éventuellement un anticoagulant tel que le polyanéthol sulfonate de sodium (SPS) et/ou un neutralisateur d'antibiotiques tel que des résines adsorbantes de cations ou du charbon activé. De manière préférée, l'hémoculture est réalisée dans un système automatisé. Les systèmes automatisés d'hémoculture sont bien connus de l'homme du métier et comprennent par exemple le Bactec® commercialisé par Becton-Dickinson et le BacT/ALERT® commercialisé par bioMérieux. Ces systèmes assurent en continu la surveillance, l'agitation et l'incubation des hémocultures. Lors de sa croissance, le microorganisme produit du CO₂ induisant une baisse du pH, qui sera détectée par l'automate à l'aide d'un sensor, soit par fluorescence, soit par réflectométrie. L'appareil avertit de tout résultat positif grâce à une alarme visuelle et/ou sonore, lorsque la concentration en microorganismes atteint une concentration seuil.

Il est à noter que les procédés selon l'invention sont particulièrement avantageux pour détecter la présence d'une enzyme d'un microorganisme dans un échantillon de sang après une étape d'hémoculture. En effet, les procédés selon l'invention permettent de réaliser la détection d'une résistance en seulement 30 min après l'étape d'hémoculture alors que les procédés de l'état de la technique ne permettent d'obtenir un résultat que 16 h minimum après l'étape d'hémoculture. Cette réduction du temps d'attente dans les procédés selon l'invention provient en partie du fait qu'il ne contient pas d'étape de culture en milieu gélosé.

### Etape de concentration

Par "concentrer" ou "concentration", on entend ici l'opération consistant à réduire le volume d'une solution contenant des microorganismes par élimination de la partie aqueuse, de sorte que sa richesse en microorganismes augmente. Les techniques de concentration sont bien connues de l'homme du métier et incluent par exemple la centrifugation et la filtration. De préférence, l'étape de concentration comprend la centrifugation de l'échantillon biologique puis l'élimination du surnageant obtenu. Typiquement, la centrifugation est réalisée à une vitesse d'environ 3000 g pendant 2 à 10 min de manière à obtenir un culot bactérien. Le surnageant issu de la centrifugation est alors éliminé. La filtration est par exemple réalisée à travers une membrane dont la taille des pores est inférieure à 1 µm (par exemple de 0,45 µm). Les microorganismes sont alors concentrés et récupérés sur la membrane. L'étape de concentration peut également être réalisée dans un tube avec gel séparateur de sérum, tel que le tube avec séparateur de sérum commercialisé par Becton Dickinson. Après centrifugation, typiquement à 1300 g pendant 10 min, les microorganismes sont alors récupérés à la surface du gel.

Par ailleurs, la concentration des microorganismes peut-être réalisée par centrifugation suite à une lyse et/ou à une agglutination des cellules sanguines et/ou une filtration permettant le passage des micro-organismes.

Les microorganismes concentrés peuvent en outre faire l'objet d'un ou plusieurs lavages en milieu tamponné (par exemple en tampon phosphate).

### Etape de lyse

Lorsque l'échantillon biologique est un échantillon contenant du sang ou des hématies, l'étape de concentration peut être précédée d'une étape de lyse des hématies présentes dans l'hémoculture. Cette étape de lyse doit permettre d'éliminer les hématies sans que les microorganismes ne soient eux-mêmes lysés. Des conditions appropriées de lyse des hématies sont bien connues de l'homme du métier et comprennent par exemple la mise en contact de l'échantillon biologique avec un tampon de lyse des globules rouges tel que le tampon de lyse RBC (Red Blood Cells lysis buffer: 150 mM NH₄Cl, 10 mM KHCO₃, 0,01 mM EDTA) pendant une période appropriée, par exemple 10 min, à une température appropriée, par exemple entre 15 et 40°C, pour permettre la lyse des globules rouges. D'autres tampons de lyse susceptibles d'être utilisés ici sont connus de l'homme du métier (Chen *et al*., (précité) ; Moreau et al., 2002, J Drug Target. 10(2):161-73).

Cette étape de lyse peut être renouvelée ou être suivie d'une étape de lavage avant l'étape de concentration.

### Etape d'agglutination

Lorsque l'échantillon biologique est un échantillon contenant du sang ou des hématies, l'étape de concentration peut être précédée d'une étape a') de préparation de l'échantillon biologique comprenant:
(i) l'agglutination des hématies, et
(ii) la séparation des hématies agglutinées des microorganismes présents dans l'échantillon.

Par "agglutiner" ou "agglutination", on entend ici l'opération consistant à faire précipiter des cellules, en particulier les cellules sanguines, et de manière préférée des hématies.

De préférence, l'agglutination est mise en oeuvre en mettant l'échantillon biologique en contact avec au moins un agent d'agglutination.

Par "agent d'agglutination", on entend ici une molécule induisant l'agglutination de cellules. L'agent d'agglutinaton selon l'invention est de préférence choisi dans le groupe constitué des lectines, incluant la concanavaline A, l'abrine, la ricine et les agglutinines telles que l'agglutinine de germe de blé et les agglutinines de soja; des acides aminés polymériques tels que la polylysine et la polyarginine; des polymères cationiques tels que le polyéthylènimine; des polymères hydrosolubles naturels ou synthétiques tels que le bromure d'hexadimethirine, le polyvinylpyrrolidone (PVP) et les polyéthylènes glycols (PEG); des polycations tels que le sulfate de protamine; les gélatines, les dextrans, le fibrinogène, l'urée, le glycérol, le chlorure de sodium et des anticorps.

De préférence, l'agent d'agglutination utilisé dans le cadre de l'invention est un polyéthylène glycol (PEG).

Des techniques d'agglutination des hématies mettant en oeuvre un agent d'agglutination tel que défini ci-dessus sont bien connues de l'homme du métier et sont par exemple décrites dans les demandes WO 03/025207 et US 4,753,776. Typiquement, l'échantillon biologique à préparer est mis en contact avec du PEG à une concentration suffisante et pendant une période suffisante pour permettre l'agglutination des hématies présentes dans l'échantillon biologique, par exemple pendant 1 à 30 min, de préférence entre 5 et 15 min.

Dans le contexte de l'invention, l'étape de "séparation des hématies agglutinées des microorganismes présents dans l'échantillon" consiste à éliminer les hématies agglutinées et conserver les microorganismes présents dans l'échantillon traité. De telles techniques de séparation sont bien connues de l'homme du métier et comprennent, par exemple, la filtration, la centrifugation et/ou la décantation.

La filtration consiste par exemple à mettre en contact l'échantillon biologique et l'agent agglutinant à la surface du filtre avant centrifugation afin de séparer les hématies agglutinées (qui restent sur le filtre) des microorganismes qui se retrouvent dans le filtrat.

La décantation consiste par exemple à mettre en contact l'échantillon biologique et l'agent agglutinant et à prélever le surnageant après agglutination et décantation.

De préférence, la séparation des hématies agglutinées est réalisée en utilisant des filtres à centrifuger.

L'étape a') de préparation de l'échantillon biologique telle que définie ci-dessus peut en outre être précédée ou suivie d'une ou de plusieurs étapes de lyse des hématies telle que définie dans le paragraphe "*Etape de lyse*" ci-dessus.

### Etape de mise en suspension

Les protocoles selon la présente invention sont caractérisés en particulier par le fait que les microorganismes, après concentration, sont mis en suspension dans une solution comprenant au moins un substrat chromogène ou fluorogène tel que décrit. Ainsi, le substrat chromogène ou fluorogène est mis en contact dans une solution avec les microorganismes concentrés, et non sur milieu solide, tel que sur milieu gélosé.

Par "mettre en suspension les microorganismes", on entend ici le fait de diminuer des agrégats bactériens par reprise dans une solution, les agrégats bactériens provenant soit d'une sédimentation naturelle, soit d'une sédimentation réalisée volontairement par exemple par centrifugation. La finalité de la mise en suspension des microorganismes selon la présente invention est de tendre vers une solution dans laquelle les microorganismes sont tous séparés les uns des autres.

La solution dans laquelle les microorganismes sont remis en suspension comprend au moins un substrat chromogène ou fluorogène tel que décrit. Elle peut contenir en outre au moins un agent de lyse. L'agent de lyse selon l'invention peut être un agent de lyse bactérien ou fongique. De préférence, l'agent de lyse lyse la paroi bactérienne ou fongique sans hydrolyser le substrat chromogène ou fluorogène.

Ledit au moins un agent de lyse peut être sélectionné dans le groupe constitué d'un détergent, d'une enzyme qui dégrade la paroi bactérienne ou fongique, d'un antibiotique actif sur la paroi bactérienne telle qu'une polymyxine, et de combinaisons de ceux-ci. Avantageusement, l'agent de lyse peut être combiné à, ou remplacé par, un moyen de lyse mécanique, par exemple des billes ou des ultrasons.

Dans le contexte de l'invention, un "détergent" ou "surfactant" est un composé chimique doté de propriétés tensioactives.

Des exemples de détergent sont bien connus de l'homme du métier et comprennent en particulier ceux décrits sur le site de la société Sigma (http://www.sigmaaldrich.com/life-science/life-science-catalog/product-catalog.html?TablePage=20964389) et les composés suivants: l'acide chénodésoxycholique; le sel de sodium d'acide chénodésoxycholique; l'acide cholique; l'acide déhydrocholique; l'acide désoxycholique; l'ester de méthyle d'acide désoxycholique; la digitonine; la digitoxigénine; l'oxyde de N,N-diméthyldodécylamine; le docusate de sodium; le sel de sodium d'acide glycochénodésoxycholique; l'hydrate d'acide glycocholique; le sel de sodium d'hydrate d'acide glycocholique; le monohydrate d'acide glycodésoxycholique; le sel de sodium d'acide glycodésoxycholique; le sel disodium 3-sulfate d'acide glycolithocholique; l'ester d'éthyle d'acide glycolithocholique; le sarkosyl, le sel de sodium de N-lauroylsarcosine; la N-lauroylsarcosine; le dodécyl sulfate de lithium; la solution de lugol; le Niaproof 4 Type 4 (*i.e.*, le sel de sodium de 7-éthyl-2-méthyl-4-undécyl sulfate); le sel de sodium d'acide 1-octanesulfonique; le sodium 1-butanesulfonate; le sodium 1-décanesulfonate; le sodium 1-dodécanesulfonate; le sodium 1-heptanesulfonate anhydre; le sodium 1-nonanesulfonate; le monohydrate de sodium 1-propanesulfonate; le sodium 2-bromoéthanesulfonate; l'hydrate de sodium cholate; le sodium choléate; le sodium désoxycholate; le monohydrate de sodium désoxycholate; le sodium dodécyl sulfate; le sodium hexanesulfonate anhydre; le sodium octyl sulfate; le sodium pentanesulfonate anhydre; le sodium taurocholate; le sodium taurodésoxycholate; le sel de sodium d'acide taurochénodésoxycholique; le monohydrate de sel de sodium d'acide taurodésoxycholique; l'hydrate de sel de sodium d'acide taurodésoxycholique; le sel de disodium d'acide 3-sulfate taurolithocholique; le sel de sodium d'acide tauroursodésoxycholique; le Trizma® dodécyl sulfate (*i.e.*, tris(hydroxyméthyl)aminométhane lauryl sulfate); l'acide ursodésoxycholique, le bromure d'alkyl triméthylammonium; le chlorure de benzalkonium; le chlorure de benzyl diméthylhexadécylammonium; le chlorure de benzyl diméthyltétradécylammonium; le bromure de benzyl dodécyldiméthylammonium; le tétrachloroiodate de benzyl triméthylammonium; le bromure de cétyl triméthylammonium; le bromure de diméthyl dioctadécylammonium; le bromure de dodécyl éthyldiméthylammonium; le bromure de dodécyl triméthylammonium; le bromure d'éthyl hexadécyldiméthylammonium; le réactif T de Girard; le bromure d'hexadécyl triméthylammonium; le N,N',N'-polyoxyéthylène(10)--N-tallow-1,3-diaminopropane; le bromure de thonzonium; le bromure de triméthyl(tétradécyl)ammonium, le BigCHAP (N,N-bis[3-(D-gluconamido)propyl]cholamide); le bis(polyéthylène glycol bis[imidazoyl carbonyl]); les alcools polyoxyéthylèniques, comme par exemple le Brij® 30 (polyoxyéthylène(4) lauryl éther), le Brij®35 (polyoxyéthylène(23) lauryl éther), le Brij® 35P, le Brij® 52 (polyoxyéthylène 2 cétyl éther), le Brij® 56 (polyoxyéthylène 10 cétyl éther), le Brij® 58 (polyoxyéthylène 20 cétyl éther), le Brij® 72 (polyoxyéthylène 2 stéaryl éther), le Brij® 76 (polyoxyéthylène 10 stéaryl éther), le Brij® 78 (polyoxyéthylène 20 stéaryl éther), le Brij® 78P, le Brij® 92 (polyoxyéthylène 2 oléyl éther); le Brij® 92V (polyoxyéthylène 2 oléyl éther), le Brij® 96V, le Brij® 97 (polyoxyéthylène 10 oléyl éther), le Brij® 98 (polyoxyéthylène(20) oléyl éther), le Brij® 58P, et le Brij® 700 (polyoxyéthylène (100) stéaryl éther); le Cremophor® EL (polyoxyéthylèneglycéroltriricinoléate 35; huile de castor polyoxyl 35); le monododécyl éther de décaéthylène glycol; le mono hexadécyl éther de décaéthylène glycol; le mono tridécyl éther de décaéthylene glycol; la N-décanoyl-N-méthylglucamine; le n-décyl alpha-D-glucopyranoside; le décyl beta-D-maltopyranoside; la digitonine; la n-dodécanoyl-N-méthylglucamide; le n-dodécyl alpha-D-maltoside; le n-dodécyl beta-D-maltoside; le monodécyl éther d'heptaéthylène glycol; le monododécyl éther d'heptaéthylène glycol; le monotétradécyl éther d'heptaéthylène glycol; le n-hexadécyl beta-D-maltoside; le monododécyl éther d'hexaéthylène glycol; le monohexadécyl éther d'hexaéthylène glycol; le monooctadécyl éther d'hexaéthylène glycol; le monotétradécyl éther d'hexaéthylène glycol; l'Igepal® CA-630 (nonylphényl-polyéthylèneglycol, (octylphénoxy)polyéthoxyéthanol, octylphényl-polyéthylène glycol); le méthyl-6-O-(N-heptylcarbamoyl)-alpha-D-glucopyranoside; le monododécyl éther de nonaéthylène glycol; la N-nonanoyl-N-méthylglucamine; le monodécyl éther d'octaéthylène glycol; le monododécyl éther d'octaéthylene glycol; le monohexadécyl éther d'octaéthylène glycol; le monooctadécyl éther d'octaéthylène glycol; le monotétradécyl éther d'octaéthylène glycol; l'octyl-beta-D-glucopyranoside; le monodécyl éther de pentaéthylène glycol; le monododécyl éther de pentaéthylène glycol; le monohexadécyl éther de pentaéthylène glycol; le monohexyl éther de pentaéthylène glycol; le monooctadécyl éther de pentaéthylène glycol; le monooctyl éther de pentaéthylène glycol; le diglycidyl éther de polyéthylène glycol; l'éther de polyéthylène glycol W-1; le polyoxyéthylène 10 tridécyl éther; le polyoxyéthylène 100 stéarate; le polyoxyéthylène 20 isohexadécyl éther; le polyoxyéthylène 20 oléyl éther; le polyoxyéthylène 40 stéarate; le polyoxyéthylène 50 stéarate; le polyoxyéthylène 8 stéarate; le polyoxyéthylène bis(imidazolyl carbonyl); le polyoxyéthylène 25 propylène glycol stéarate; la saponine d'écorce de quillaja; les esters d'acide gras sorbitane, par exemple le Span® 20 (sorbitane monolaurate), le Span® 40 (sorbitane monopalmitate), le Span® 60 (sorbitane monostéarate), le Span® 65 (sorbitane tristéarate), le Span® 80 (sorbitane monooléate), et le Span® 85 (sorbitane trioléate); les différents alkyl éthers de polyéthylène glycols, comme par exemple le Tergitol® Type 15-S-12, le Tergitol® Type 15-S-30, le Tergitol® Type 15-S-5, le Tergitol® Type 15-S-7, le Tergitol® Type 15-S-9, le Tergitol® Type NP-10 (nonylphénol éthoxylate), le Tergitol® Type NP-4, le Tergitol® Type NP-40, le Tergitol® Type NP-7, le Tergitol® Type NP-9 (nonylphénol polyéthylène glycol éther), le Tergitol® MIN FOAM IX, le Tergitol® MIN FOAM 2X, le Tergitol® Type TMN-10 (polyéthylène glycol triméthylnonyl éther), le Tergitol® Type TMN-6 (polyéthylène glycol triméthylnonyl éther), le Triton® 770, le Triton® CF-10 (benzyl-polyéthylène glycol tert-octylphényl éther), le Triton® CF-21, le Triton® CF-32, le Triton® DF-12, le Triton® DF-16, le Triton® GR-5M, le Triton® N-42, le Triton® N-57, le Triton® N-60, le Triton® N-101 (polyéthylène glycol nonyl phényl éther; polyoxyéthylène ramifié nonyl phényl éther), le Triton® QS-15, le Triton® QS-44, le Triton® RW-75 (polyéthylène glycol 260 mono(hexadécyl/octadécyl) éther et 1-octadécanol), le Triton® SP-135, le Triton® SP-190, le Triton® W-30, le Triton® X-15, le Triton® X-45 (polyéthylène glycol 4-tert-octylphényl éther; 4-(1,1,3,3-tétraméthylbutyl)phényl-polyéthylène glycol), le Triton® X-100 (t-octylphénoxypolyéthoxyéthanol; polyéthylène glycol tert-octylphényl éther; 4-(1,1,3,3-tétraméthylbutyl)phényl-polyéthylène glycol), le Triton® X-102, le Triton® X-114 (polyéthylène glycol tert-octylphényl éther; (1,1,3,3-tétraméthylbutyl)phényl-polyéthylène glycol), le Triton® X-165, le Triton® X-305, le Triton® X-405 (polyoxyéthylène(40) isooctylcyclohéxyl éther; polyéthylène glycol tert-octylphényl éther), le Triton® X-705-70, le Triton® X-151, le Triton® X-200, le Triton® X-207, le Triton® X-301, le Triton® XL-80N, et le Triton® XQS-20; le tétradécyl-beta-D-maltoside; le tétraéthylène glycol monodécyl éther; le tétraéthylène glycol monododécyl éther; le tétraéthylène glycol monotétradécyl éther; le triéthylène glycol monodécyl éther; le triéthylène glycol monododécyl éther; le triéthylène glycol monohexadécyl éther; le triéthylène glycol monooctyl éther; le triéthylène glycol monotétradécyl éther; les esters d'acide gras polyoxyéthylène sorbitane, par exemple le TWEEN® 20 (polyéthylène glycol sorbitane monolaurate), le TWEEN® 20 (polyoxyéthylène (20) sorbitane monolaurate), le TWEEN® 21 (polyoxyéthylène (4) sorbitane monolaurate), le TWEEN® 40 (polyoxyéthylène (20) sorbitane monopalmitate), le TWEEN® 60 (polyéthylène glycol sorbitane monostéarate; polyoxyéthylène (20) sorbitane monostéarate), le TWEEN® 61 (polyoxyéthylène (4) sorbitane monostéarate), le TWEEN® 65 (polyoxyéthylène (20) sorbitane tristéarate), le TWEEN® 80 (polyéthylène glycol sorbitane monooléate; polyoxyéthylène (20) sorbitane monooléate), le TWEEN® 81 (polyoxyéthylène (5) sorbitane monooléate), et le TWEEN® 85 (polyoxyéthylène (20) sorbitane trioléate); le tyloxapol; le n-undécyl beta-D-glucopyranoside, le CHAPS (3-[(3-cholamidopropyl)diméthylammonio]-1-propanesulfonate); le CHAPSO (3-[(3-cholamidopropyl)diméthylammonio]-2-hydroxy-1-propanesulfonate); le N-dodécylmaltoside; l'alpha-dodécyl-maltoside; le beta-dodécyl-maltoside; le sel interne de 3-(décyldiméthylammonio)propanesulfonate (SB3-10 le sel interne de 3-(décyldiméthylammonio)propanesulfonate (SB3-10), le sel interne de 3-(dodécyldiméthylammonio)propanesulfonate (SB3-12), le sel interne de 3-(N,N-diméthylmyristylammonio)propanesulfonate (SB3-14), le sel interne de 3-(N,N-diméthylpalmitylammonio)propanesulfonate (SB3-16), le sel interne de 3-(N,N-diméthyloctadécylammonio)propanesulfonate (SB3-18); le MEGA-8; le MEGA-9; le MEGA-10; le méthylheptylcarbamoyl glucopyranoside; la N-nonanoyl N-méthylglucamine; l'octyl-glucopyranoside; l'octyl-thioglucopyranoside; l'octyl-beta-thioglucopyranoside; le 3-[N,N-diméthyl(3-myristoylaminopropyl)ammonio]propanesulfonate ou l'amidosulfobétaine-14 (ASB-14); l'amidosulfobétaine-16 (ASB-16) ; l'EMPIGEN® BB ; le Cymal-1, le Cymal-2, le Cymal-5, le Cymal-6 et l'acide désoxycholique.

De préférence, dans les procédés selon l'invention, le détergent est sélectionné dans le groupe constitué du CHAPS, du Tween 20, du Tween 80, du c7bzo, de l'octylglucoside, de l'octylthioglucopyranoside, de l'ASB-14, du SB3-10, du sodium dodécyl sulfate, de la digitonine, du sarkosyl et du tergitol.

L'agent de lyse peut également être une enzyme qui dégrade la paroi bactérienne telle que le lysozyme, la lysostaphine, l'achromopeptidase, la mutanolysine, la labiase, la chitinase, la glucanase, la glucosaminidase, la muramidase, la transglycosylase lytique, l'amidase et l'endopeptidase.

L'agent de lyse peut être un agent causant la formation de pores dans la paroi bactérienne ou la membrane cellulaire comme par exemple une polymyxine, en particulier une polymyxine A, B, C, D, E (ou colistine), F, K, M, P, S ou T. Les polymyxines sont des antibiotiques peptidiques cycliques qui agissent comme des détergents cationiques et s'insèrent parmi les phospholipides de la paroi bactérienne. Il peut s'agir également de β-lactames, de glycopeptides, de fosfomycine, de cyclosérine, de bacitracine, d'acide fusidique ou de néomycine.

Lors de l'étape b1) de mise en suspension, le substrat chromogène ou fluorogène peut en outre être mis en présence d'au moins un inhibiteur de β-lactamase. La solution dans laquelle les microorganismes sont remis en suspension peut donc comprendre en outre au moins un inhibiteur de β-lactamase, en particulier au moins un inhibiteur de β-lactamase sélectionné dans le groupe constitué d'un inhibiteur de céphalosporinase, un inhibiteur de sérine β-lactamase, un chélateur de métal et un inhibiteur de BLSE.

Des exemples d'inhibiteurs de β-lactamase sont bien connus de l'homme du métier et incluent en particulier la cloxacilline, le composé syn2190, l'aztreonam, l'acide boronique et des dérivés de celui-ci tels que l'acide 3-aminophényl boronique, l'acide phényl boronique, l'acide benzo(b)thiophene-2-boronique, l'acide *meta-*carboxyphenylboronique ; l'acide clavulanique, des sels de l'acide clavulanique, le tazobactam et le sulbactam ; l'acide dipicolinique (DPC), le diéthyldithiocarbamate (DEDTC), la N,N,N',N'-tetrakis-(2-pyridylmethyl)-ethylenediamine (TPEM), l'acide éthylène diamine tetra acétique (EDTA), l'acide 2,3-dimercapto-1-propane sulfonique (DMPS) et la 1,10-phenanthroline ; la ceftazidime, un sel de la ceftazidime, la cefotaxime et un sel de la cefotaxime ; le monobactame sidérophore BAL30072, la 2-amino-4-thiazolyl methoxyimino-pénicilline (ATMO-pénicilline) ; le C-6-mercaptomethyl penicillinate; le méthylidène pénème BRL 42715 ; le méthylidène pénème BLI-489; des alkylidène pénèmes; des méthylidène pénèmes tri- et bicycliques; le carbapénème tricyclique LK-157; le 1-b-methylcarbapénème 225; le sulfone de péname Ro 48-1220; le sulfone de péname LN-1-255; le sel de sodium de trans-7-oxo-6-(sulfooxy)-1,6-diazabicyclo[3.2.1]octan-2-carboxamide (NXL104); les phosphonates et des dérivés de ceux-ci tels que les cyclic acyl phosphonates ; les pénicillines telles que JDB/LN-I-255 ; les céphalosporines sulfoniques comme JBB/DVR-II-214. Il est connu que le choix du ou des inhibiteur(s) utilisable(s) peut dépendre du type d'enzyme que l'on souhaite détecter (WO2009/051838).

De préférence, les microorganismes sont laissés en suspension dans la solution comprenant un substrat chromogène ou fluorogène selon l'invention à une température et pendant une période appropriée pour permettre l'hydrolyse du substrat par l'enzyme et détecter une éventuelle libération du chromophore ou fluorophore. De telles conditions sont bien connues de l'homme du métier et sont fonctions du substrat chromogène ou fluorogène utilisé. Typiquement, les microorganismes sont laissés à incuber pendant 5 min à 2 h, à une température comprise entre 15 et 42°C. La libération du chromophore ou fluorophore, qui se caractérise par un changement de couleur du substrat chromogène ou d'émission de fluorescence du substrat fluorogène, peut alors être directement observée en solution.

Les procédés selon l'invention ont l'avantage de diminuer fortement le temps au bout duquel il est possible de détecter la présence d'une enzyme d'un microorganisme dans un échantillon biologique. Ce gain de temps provient en particulier du fait que les procédés selon l'invention ne comprennent pas d'étape de culture des microorganismes dans un milieu sélectif contenant un antibiotique spécifique. En particulier, ils ne comprennent pas d'étape de sélection ni d'induction des microorganismes sur milieu gélosé.

Lorsque l'échantillon biologique est un échantillon contenant du sang ou des hématies, le procédé de détection selon l'invention peut comprendre en outre une étape de lyse des hématies présentes dans l'échantillon biologique avant l'étape de mise en suspension, comme décrit dans la section "*Etape de lyse*" ci-dessus.

### Procédé de préparation d'un échantillon d'hémoculture

La présente demande décrit également un procédé de préparation *in vitro* d'un échantillon d'hémoculture comprenant des microorganismes, comprenant les étapes consistant à:
A) lyser ou agglutiner les hématies présentes dans l'échantillon d'hémoculture sans lyser les microorganismes présents dans l'échantillon d'hémoculture,
B) séparer les microorganismes présents dans l'échantillon d'hémoculture des hématies lysées ou agglutinées à l'étape A), et
C) éventuellement laver les microorganismes de l'échantillon d'hémoculture séparés à l'étape B).

De préférence, le procédé de préparation ci-dessus permet d'obtenir des microorganismes sur lesquels il est possible d'appliquer l'étape a1) des procédés de détection tels que définis ci-dessus.

L'étape A) de lyse des hématies peut être typiquement mise en oeuvre comme indiqué ci-dessus dans le paragraphe "*Etape de lyse*", en mettant en contact l'échantillon d'hémoculture avec un tampon de lyse des globules rouges tel que le tampon de lyse RBC, pendant une période appropriée, par exemple entre 1 min et 10 min, à une température appropriée, par exemple à température ambiante ou à 37°C, pour permettre la lyse des hématies.

L'étape A) d'agglutination des hématies peut être typiquement mise en oeuvre comme indiqué ci-dessus dans le paragraphe "*Etape d'agglutination"* en mettant en contact l'hémoculture avec au moins un agent d'agglutination tel que défini ci-dessus.

L'étape B) de séparation des microorganismes présents dans l'échantillon d'hémoculture peut être mise en oeuvre par toute technique appropriée bien connue de l'homme du métier. L'étape de séparation des microorganismes peut en particulier être mise en oeuvre par centrifugation, filtration ou prélèvement de la phase aqueuse, par exemple, après décantation. De préférence, l'étape de séparation des microorganismes est mise en oeuvre par centrifugation.

L'étape optionnelle de lavage peut être typiquement mise en oeuvre par resuspension des microorganismes séparés dans un tampon de lavage, tel que le tampon de lyse RBC, incubation pendant une période appropriée, à une température appropriée, et nouvelle séparation des microorganismes, par exemple par centrifugation.

### Procédé de détection in vitro de microorganismes

Comme indiqué ci-dessus, dans les procédés de détection *in vitro* d'enzymes, les enzymes à détecter sont produites par des microorganismes particuliers. La présence de l'enzyme à détecter signifie donc que le microorganisme produisant cette enzyme est présent dans l'échantillon biologique.

Par conséquent, la présente demande décrit également un procédé de détection *in vitro* de microorganismes à partir d'un échantillon biologique mettant en oeuvre le procédé de détection d'enzyme tel que défini ci-dessus.

En particulier, est décrit ici un procédé de détection *in vitro* d'un microorganisme à partir d'un échantillon biologique comprenant les étapes consistant à :
a1) concentrer les microorganismes présents dans l'échantillon biologique, éventuellement après une étape a0) de culture des microorganismes telle que définie ci-dessus;
b1) mettre en suspension les microorganismes concentrés à l'étape a1) dans une solution comprenant au moins un substrat chromogène ou fluorogène susceptible de libérer un chromophore ou un fluorophore après hydrolyse par une enzyme du microorganisme à détecter tel que défini ci-dessus;
c1) détecter l'éventuelle libération du chromophore ou du fluorophore obtenue à l'étape b1);
la libération du chromophore ou du fluorophore détectée à l'étape c1) étant indicative de la présence du microorganisme à détecter.

La détection de la libération du chromophore ou du fluorophore du substrat chromogène ou fluorogène est donc indicatif de la présence de microorganismes exprimant l'enzyme spécifique du substrat chromogène ou fluorogène.

Les exemples suivants illustrent l'invention sans en limiter la portée.

### Exemple 1 : Détection de β-lactamase conférant aux microorganismes la produisant une résistance aux céphalosporines de 3^{ème} génération, directement à partir d'urines, sans étape de croissance bactérienne

Cet exemple montre qu'il est possible d'identifier des entérobactéries résistantes aux céphalosporines de 3^{ème} génération (C3G) directement à partir d'urine.

Deux urines ont été collectées de sujets sains (un homme et une femme). Ces urines ont chacune été inoculées de façon séparée par différentes charges de deux souches d'*Escherichia coli* (T434 = résistante aux C3G ayant une activité β-lactamase importante ; ATCC 25922 = sensible).

Brièvement, 1 ml d'urine a été inoculé avec 1, 10 ou 100 µL d'une suspension bactérienne à 0,5 McFarland (McF). Les tubes ont été centrifugés à 3000 g ou 6000 g éventuellement avec des lavages.

Sur le culot bactérien obtenu ont été ajoutés 20 µL d'une solution comprenant 0,8 g/L de substrat HMRZ-86 et 20 µL de CHAPS à 20 g/L. Après mélange au vortex 10 secondes, le tube a été laissé 30 min à température ambiante.

Les inventeurs ont observé une réaction positive pour l'inoculum le plus fort. Ainsi, un virage du jaune vers l'orangé était visible avec la souche résistante quand son nombre atteignait 6.10⁶ ufc/mL alors qu'aucun virage n'était visible avec la souche sensible.

### Exemple 2 : Détection de β-lactamase conférant aux microorganismes la produisant une résistance aux céphalosporines de 3^{ème} génération, avec une étape de croissance bactérienne

Ce test a été réalisé en microplaque.

Chaque puits contenait
- 50 µL de HMRZ-86 dissous dans du milieu TCS 60 g/L tamponné (tampon phosphate 0,2 M pH 6)
- 40 µL de CHAPS ou H₂O
- 10 µL d'une suspension bactérienne dense (5 McF, > 10⁸ ufc/ml).

Les microplaques ont été observées toutes les heures afin de détecter un éventuel virage de couleur.

Au bout de 3 h, par exemple, sur un panel de 8 souches résistantes et 4 souches sensibles, avec 0,4 g/L de HMRZ-86 et entre 10 et 20 g/L de CHAPS, et un inoculum fort, les résultats présentés dans le **tableau 2** ont été obtenus.

**Tableau 2**

| | Eau | | CHAPS | |
|---|---|---|---|---|
| | Souches résistantes | Souches sensibles | Souches résistantes | Souches sensibles |
| Virage « significatif » | 3 | | 5 | |
| Virage douteux | 1 | | | |
| Absence de virage | 4 | 4 | 3 | 4 |

Au bout de 3h30, dans un autre exemple, sur un panel de 8 souches résistantes et 4 souches sensibles avec 0,4 g/L de HMRZ-86, en présence ou non de 5 g/L de CHAPS et à partir d'un inoculum fort, les résultats présentés dans le **tableau 3** ont été obtenus.

**Tableau 3**

| | Absence de CHAPS | | Présence de CHAPS | |
|---|---|---|---|---|
| | Souche résistante | Souche sensible | Souche résistante | Souche sensible |
| Virage « significatif » | 4 | | 6 | |
| Virage douteux | 1 | 1 | | |
| Absence de virage | 3 | 3 | 2 | 4 |

Ainsi, la présence de CHAPS favorise la réaction dans le cas d'une étape de croissance bactérienne préalable.

### Exemple 3 : Détection de β-lactamase conférant aux microorganismes la produisant une résistance aux céphalosporines de 3^{ème} génération, directement à partir d'hémoculture

Cet exemple montre qu'il est possible d'identifier des entérobactéries résistantes aux céphalosporines de 3^{ème} génération (C3G) directement à partir d'hémoculture.

Pour étudier la possibilité de détection des souches résistantes aux C3G dans les hémocultures, le protocole adopté a été le suivant (remarque : des variantes au protocole n'entrainant pas de variation dans le rendu des résultats, sont notées entre crochets. Elles ont été appliquées sur 2 souches, une sensible et une résistante. Un virage de couleur a été observé pour la souche résistante et aucun virage n'a été observé pour la souche sensible) :

7,5 mL de milieu issu de flacon d'hémoculture (bacTAlert ; réf 259791 ou 259793) ont été additionnés de 2,5 mL de sang de mouton défibriné. 25 µL d'une suspension bactérienne à 0,5 McF ont été ajoutés. Après homogénéisation au vortex et incubation pendant 18-20 h à 37°C, 500 µL ont été prélevés. 0,5 mL [ou 1mL] d'une solution de lyse de globules rouges modifiée contenant notamment du NH₄Cl mais ne contenant pas d'EDTA (SL) ont été ajoutés. Après homogénéisation au vortex, le tube a été incubé 10 min à 37°C, [ou 1 min à 37°C ou 1 min à température ambiante]. Un culot, récupéré suite à une centrifugation à 750 g pendant 5 min [ou 2 min], a été resuspendu dans 200 µL de SL et laissé 2 min à température ambiante. 1 mL de PBS 0,1 M a alors été ajouté. Le tube a de nouveau été centrifugé à 750 g pendant 5 min [ou 2 min], et le culot a été repris dans une solution comprenant 50 µL de substrat HMRZ-86 (1,2 g/L) et 50 µL de CHAPS (30 g/L). Après homogénéisation au vortex, le tube a été mis à incuber 15 min à température ambiante. Après centrifugation à 750 g pendant 5 min [ou 2 min], le changement de couleur a été observé.

Le test a été réalisé sur 4 souches dont 3 souches résistantes aux C3G (dont 2 métallo-β-lactamases dont la réaction était inhibée en présence du milieu de culture), et une sensible.

Un essai a été réalisé sur les deux milieux d'hémoculture (aérobie et anaérobie) sur un panel plus large. En milieu aérobie, 17 souches résistantes aux C3G sur 18 ont répondu positivement au test et 6 souches sensibles sur 8 n'ont pas montré de virage significatif de coloration. En milieu anaérobie, 16 souches résistantes aux C3G sur 18 ont répondu positivement au test et 6 souches sensibles sur 8 n'ont pas montré de virage significatif de coloration.

Des essais ont été de même réalisés à partir de flacons de milieu pour hémoculture supplémentés de sang total humain.

Différents protocoles ont été suivis :

7,5 mL de milieu issu de flacon d'hémoculture (bacTAlert ; réf 259791 ou 259793) ont été additionnés de 2,5 mL de sang total humain. 25 µL d'une suspension bactérienne à 0,5 McF ont été ajoutés. Après homogénéisation au vortex, le tube a été incubé 18-20 h à 37°C.

### Protocole de lyse

0,5 ml d'hémoculture ont été prélevés et additionnés de 0,5 mL de tampon de lyse. Le mélange a été laissé en contact pendant 10 min puis centrifugé 2 min à 750 g. Le culot a subi encore deux fois le même protocole (ajout du tampon de lyse puis centrifugation). Il a ensuite été repris dans une solution comprenant 50 µL de substrat HMRZ-86 (1,2 g/L) et 50 µL de CHAPS (30 g/L). Après homogénéisation au vortex, le tube a été incubé 15 min à température ambiante. Tout changement de couleur a été noté après centrifugation 2 min à 750 g.

### Protocole « agglomération - filtration sur filtre à centrifuger »

50 mg de PEG ont été déposés sur un filtre à centrifuger type « Filtres pour centrifugation Millipore Ultrafree MC centrifugal filter units 5µm ». 0,5mL d'hémoculture ont été prélevés et déposés sur le filtre et laissés en contact avec le PEG pendant 5 min. Après une centrifugation 5 min à 5500 g (qui a permis la filtration) et élimination du surnageant au dessus du filtre, le filtrat a été traité avec 1 mL de tampon de lyse pendant 10 min. Le tube a été centrifugé 5 min à 5500 g, le surnageant a été éliminé.

Le culot a ensuite été repris dans une solution comprenant 50 µL de substrat HMRZ-86 (0,8 g/L) et 50 µL de CHAPS (20 g/L). Après homogénéisation au vortex, le tube a été incubé 15 min à température ambiante, puis tout changement de couleur a été noté.

### Protocole « agglomération - décantation »

1 ml d'hémoculture a été prélevé et additionné de 200 mg de PEG. Le mélange a été laissé décanter pendant 15 min. 500 µL de surnageant ont été placés dans un autre tube. Après une centrifugation 5 min à 5500 g et élimination du surnageant, le culot a été traité avec 1 mL de tampon de lyse, et après 10 min il a subi une nouvelle centrifugation 5 min à 5500 g.

Le culot a ensuite été repris dans une solution comprenant 50 µL de substrat HMRZ-86 (0,8 g/L) et 50 µL de CHAPS (20 g/L). Après homogénéisation au vortex suivie d'une incubation 15 min à température ambiante, tout changement de couleur a été noté.

L'ensemble de ces trois protocoles permet de limiter la coloration rouge due à l'hémoculture et l'identification rapide des C3GR.

Ils ont été appliqués à une souche C3GR et une souche C3GS : le test avec la souche C3GR montrait une coloration rouge vif alors que le test avec la souche C3GS restait jaune orangé.

### Exemple 4 (hors invention) : Détection de l'activité glycosidase de Candida tropicalis (substrat : 5-bromo 4-chloro 3-indolyl α-glucoside) d'Enterococcus faecalis (substrat : 5-bromo 4-chloro 3-indolyl β-glucoside) et de l'activité estérase de Pseudomonas aeruginosa ou de Salmonella enteritidis (substrat : 5-bromo 4-chloro 3-indolyl butyrate) directement à partir d'urine

Des urines provenant de sujets sains ont été artificiellement contaminées. Pour cela, une suspension bactérienne à 0,5 McF (environ 10⁷⁻⁸ ufc/ml) a été préparée dans de l'eau physiologique. Cette suspension a été centrifugée pendant 5 min à 6000 g et le surnageant a été éliminé. Le culot a ensuite été repris dans 1 mL d'urine de manière à obtenir une concentration d'environ 10⁷⁻⁸ ufc/ml d'urine. L'urine contaminée a été incubée pendant 15 à 30 min. Elle a ensuite été centrifugée pendant 5 min à 6000 g et le surnageant a été éliminé. Le culot a été repris dans une solution comprenant 50 µL de substrat chromogénique 2X et 50 µL de détergent 2X. Le virage de couleur a été observé après 30 min d'incubation à température ambiante.

Les résultats obtenus dans le test de détection d'activité glycosidase sont présentés dans les **tableaux 4 et 5.**

**Tableau 4**

| | *Candida tropicalis* ATCC 750 |
|---|---|
| Substrat | X-α-glucoside |
| Temps de lecture | 30 min |
| Tampon phosphate 0,1 M pH 7 (Tp) | Blanc |
| Tp + Digitonine 0,5 g/L | **Bleu** |
| Tp + OTG 5 g/L | **Bleu** |

| | |
|---|---|
| (OTG = Octyl-β-thioglucopyranoside; X=5-bromo 4-chloro 3-indolyl). | |

**Tableau 5**

| | *Enterococcus faecalis* ATCC 29212 |
|---|---|
| Substrat | X-β-glucoside |
| Temps de lecture | 30 min à TA |
| Tampon phosphate 0,1 M pH 7 | Reflet bleu / bleu clair |

Les résultats obtenus dans le test de détection d'activité estérase sont présentés dans les **tableaux 6** et **7.**

**Tableau 6**

| | *P. aeruginosa* RDC 45 |
|---|---|
| Substrat | X-butyrate |
| Temps de lecture | 45 min à 37°C |
| Tampon phosphate 0,1 M p H7 (Tp) | Blanc |
| Tp + PolyB 250 mg/L | **Bleu** |
| Tp + CHAPS 10 g/L | Blanc |
| Tp + CHAPS + PolyB | **Bleu** |

| | |
|---|---|
| (polyB = polymyxine B) | |

**Tableau 7**

| | *Salmonella enteritidis* ATCC 13076 |
|---|---|
| Substrat | X-butyrate |
| Tampon | Tampon phosphate 0,1 M pH 7 (Tp) |
| Temps de lecture | 1 h à TA |
| Tp +PolyB 250 mg/L | Reflet bleu (RB) / bleu clair |
| Tp + CHAPS 10 g/L | RB |
| Tp + Tween 20 0,2% | RB |
| Tp + CHAPS + PolyB | RB / bleu clair |
| Tp + Tween 20 + PolyB | RB / bleu clair |

L'influence de la température et du temps d'incubation a été testée (voir **tableau 8).**

**Tableau 8**

| | *P. aeruginosa* RDC 45 | | |
|---|---|---|---|
| Substrat | X-butyrate | | |
| Temps de lecture | 1 h à TA | 45 min à 37°C | 1h15 à 37°C |
| Témoin négatif sans bactérie en Tp (Tampon phosphate 0,1 M pH 7) | ND | Blanc | Blanc |
| Tp | ND | Blanc | Reflet bleu (RB) |
| Tp + CHAPS 10g/L | Blanc | Blanc | **RB** |
| Tp + PolyB 250mg/L | **RB-** | **Bleu clair** | **Bleu** |
| Tp + CHAPS + PolyB | **RB-** | **Bleu clair** | **Bleu *-*** |

| | | | |
|---|---|---|---|
| ND : non déterminé | | | |

### Exemple 5 : Détection de β-lactamase conférant aux microorganismes la produisant une résistance aux céphalosporines de 3^{ème} génération, directement à partir d'hémocultures positives à bacilles à Gram négatif.

Cet exemple montre qu'il est possible de détecter la résistance aux céphalosporines de 3^{ème} génération (C3G) chez des entérobactéries ou autres bacilles à Gram négatif comme les *Pseudomonas* directement à partir d'hémoculture.

L'étude a été réalisée en prospectif à partir d'hémocultures positives à bacilles à Gram négatif aérobies. Le protocole ci-dessous a été réalisé le jour-même où les hémocultures ont été détectées comme positives par l'automate (BacT/ALERT de Biomérieux).

### Protocole de lyse

0,5 ml d'hémoculture a été prélevé et additionné de 0,5 mL de tampon de lyse. Le mélange a été agité brièvement au vortex puis a été laissé en contact pendant 10 min à température ambiante. Une étape de centrifugation (2 min à 3000 rpm) a ensuite été réalisée et le surnageant a été éliminé. Le culot a ensuite subi par deux fois les étapes suivantes : ajout de 1 mL de tampon de lyse, brève agitation au vortex, attente de 10 min, puis centrifugation 2 min à 3000 rpm et élimination du surnageant. Le culot a ensuite été repris dans une solution comprenant 50 µL de substrat HMRZ-86 (1,2 g/L) et 50 µL de CHAPS (30 g/L). Après homogénéisation au vortex, le tube a été incubé 15 min à température ambiante. Tout changement de couleur a été noté après centrifugation 2 min à 3000 rpm.

Les souches détectées sont détaillées dans le **tableau 9.**

**Tableau 9**

| | **Souches sensibles (C3GS)** | **Souches résistantes (C3GR)** |
|---|---|---|
| *Acinetobacter baumannii* | 1 | - |
| *Citrobacter freundii* | 1 | - |
| *Escherichia coli* | 10 | 3 |
| *Klebsiella oxytoca* | - | 1 |
| *Klebsiella pneumoniae* | 1 | - |
| *Pseudomonas aeruginosa* | 1 | - |
| *Yersinia enterocolitica* | 1 | - |
| **TOTAL** | **15** | **4** |

Les résultats sont présentés dans le **tableau 10.**

**Tableau 10**

| | **Souches résistantes** | **Souches sensibles** |
|---|---|---|
| Virage « significatif » | 4 | 1 |
| Virage douteux | - | - |
| Absence de virage | - | 14 |

Cet exemple montre donc que le protocole de lyse permet la détection de β-lactamase conférant aux bacilles à Gram négatif aérobies la produisant une résistance aux céphalosporines de 3^{ème} génération directement à partir d'hémocultures positives. A part une souche C3GS qui a été détectée comme "faux positif", le test réalisé avec les souches C3GS montrait une coloration jaune clair à jaune orangé alors que le test avec les souches C3GR virait de jaune au rouge vif.

## Revendications

1. Procédé de détection *in vitro* d'une enzyme d'un microorganisme résistant aux céphalosporines de 3^{ème} génération à partir d'un échantillon biologique, ladite enzyme à détecter étant sélectionnée dans le groupe constitué par les β-lactamases à spectre étendu, les céphalosporinases et les carbapénémases, ledit procédé comprenant les étapes consistant à :
a1) concentrer les microorganismes présents dans l'échantillon biologique, éventuellement après une étape a0) de culture des microorganismes;
b1) mettre en suspension les microorganismes concentrés à l'étape a1) dans une solution comprenant au moins un substrat chromogène susceptible de libérer un chromophore après hydrolyse par l'enzyme à détecter, ledit substrat chromogène étant le composé HMRZ-86 ((7R)-7-[2-(aminothiazol-4-yl)-(z)-2-(1-carboxy-1-methylethoxyimino)acetamido]-3-(2,4-dinitrostyryl)-3-cephem-4-carboxylic acid thrifluoroacetate, isomère E) ou un sel de celui-ci;
c1) détecter l'éventuelle libération du chromophore obtenue à l'étape b1);
la libération du chromophore détectée à l'étape c1) étant indicative de la présence de l'enzyme à détecter et
ledit procédé comprenant en outre, lorsque l'échantillon biologique est un échantillon contenant du sang ou des hématies:
- une étape de lyse des hématies présentes dans l'échantillon biologique avant l'étape b1) de mise en suspension ou avant l'étape a1) de concentration, et/ou
- une étape a') de préparation de l'échantillon biologique avant l'étape a1) de concentration de microorganismes, l'étape a') comprenant :
(i) l'agglutination des hématies, et
(ii) la séparation des hématies agglutinées des microorganismes présents dans l'échantillon.

2. Procédé selon la revendication 1, dans lequel l'échantillon biologique est un échantillon de sang ou un échantillon d'urine.

3. Procédé selon la revendication 1 ou 2, dans lequel l'échantillon biologique est un échantillon de sang et l'étape a0) de culture est une étape d'hémoculture.

4. Procédé selon l'une quelconque des revendications 1 à 3, dans lequel à l'étape
b1) le substrat chromogène est mis en présence d'au moins un inhibiteur de β-lactamase sélectionné dans le groupe constitué d'un inhibiteur de céphalosporinase, un inhibiteur de sérine β-lactamases, un chélateur de métal et un inhibiteur de β-lactamase à spectre étendu.

5. Procédé selon l'une quelconque des revendications 1 à 4, dans lequel l'étape a1) de concentration comprend la centrifugation de l'échantillon biologique puis l'élimination du surnageant obtenu.

## Patentansprüche

1. *In-vitro*-Verfahren zur Detektion von einem Enzym eines Mikroorganismus, der gegen Cephalosporine der 3. Generation resistent ist, aus einer biologischen Probe, wobei besagtes zu detektierendes Enzym ausgewählt ist aus der Gruppe bestehend aus Breitband-β-Laktamasen, Cephalosphorinasen und Carbapenemasen, wobei besagtes Verfahren folgende Schritte umfasst:
a1) Konzentrieren der Mikroorganismen, die in der biologischen Probe vorhanden sind, gegebenenfalls nach einer Phase a0) der Mikroorganismenkultur;
b1) Suspendieren der in Schritt a1) konzentrierten Mikroorganismen in einer Lösung, die mindestens ein chromogenes Substrat enthält, das geeignet ist, nach der Hydrolyse durch das zu detektierende Enzym ein Chromophor freizusetzen, wobei besagtes chromogenes Substrat die Zusammensetzung HMRZ-86 ((7R)-7-[2-(Aminothiazol-4-yl)-(z)-2-(1-carboxy-1-methylethoxyimino)acetamido]-3-(2,4-dinitrostyryl)-3-cephem-4 carboxylsäuretrifluoracetat, Isomer E), oder ein Salz hiervon, ist;
c1) Detektieren der eventuellen Freisetzung des Chromophors, die in Schritt b1) erhalten wurde;
wobei die Freisetzung des Chromophors, die in Schritt c1) detektiert wird die Gegenwart des zu detektierenden Enzyms anzeigt und
besagtes Verfahren des Weiteren, wenn die biologische Probe eine Blut enthaltende Probe oder Erythrozyten ist, umfasst:
- einen Schritt der Lyse von Erythrozyten, die in der biologischen Probe vorhanden sind, vor dem Suspendieren in Schritt b1) oder vor dem Konzentrieren in Schritt a1), und/oder
- einen Schritt a') der Vorbereitung der biologischen Probe vor dem Konzentrieren der Mikroorganismen in Schritt a1), wobei der Schritt a') umfasst:
(i) die Agglutination von Erythrozyten;
(ii) die Trennung von agglutinierten Erythrozyten von den in der Probe vorhandenen Mikroorganismen.

2. Verfahren nach Anspruch 1, bei dem besagte biologische Probe eine Blutprobe oder Urinprobe ist.

3. Verfahren nach einem der Ansprüche 1 oder 2, bei dem besagte biologische Probe eine Blutprobe ist und die Phase a0) der Kultur eine Blutkultur ist.

4. Verfahren nach einem der Ansprüche 1 bis 3, bei dem im Schritt b1) das chromogene Substrat mindestens einem β-Laktamase-Inhibitor, der ausgewählt ist aus der Gruppe bestehend aus einem Cephalosporinase-Inhibitor, einem Serin-β-Laktamase-Inhibitor, einem Metallchelator, und einem Breitband-β-Laktamase-Inhibitor, ausgesetzt wird.

5. Verfahren gemäß einem der Ansprüche 1 bis 4, bei dem die Konzentrierung in Schritt a1) die Zentrifugation der biologischen Probe und dann die Beseitigung des erhaltenen Überstandes umfasst.

## Claims

1. An *in vitro* method for detecting an enzyme of a microorganism which is resistant to 3^{rd} generation cephalosporins, from a biological sample, said enzyme to be detected being chosen from the group formed by extended spectrum β-lactamases, cephalosporinases and carbapenemases, said method comprising the steps consisting of:
a1) concentrating the microorganisms present in the biological sample, optionally after a a0) culture step of the microorganisms;
b1) suspending the microorganisms concentrated at step a1) in a solution comprising at least one chromogenic substrate capable of releasing a chromophore after hydrolysis by the enzyme to be detected, said chromogenic substrate being the compound HMRZ-86 ((7R)-7-[2-(aminothiazol-4-yl)-(z)-2-(1-carboxy-1-methylethoxyimino)acetamido]-3-(2,4-dinitrostyryl)-3-cephem-4-carboxylic acid thrifluoroacetate, E-isomer) or a salt thereof;
c1) detecting potential release of the chromophore obtained at step b1);
the release of the chromophore detected at step c1) indicating the presence of the enzyme to be detected and
said process further comprising, when the biological sample is a sample containing blood or red blood cells:
- a step of lysing red blood cells present in the biological sample before the suspending step b1) or before the concentration step a1), and/or
- a step a') of preparing the biological sample before the microorganisms concentration step a1), the step a') comprising: :
(i) agglutination of the red blood cells, and
(ii) separation of the agglutinated red blood cells from the microorganisms present in the sample.

2. The method according to claim 1 wherein the biological sample is a blood sample or urine sample.

3. The method according to claim 1 or 2 wherein the biological sample is a blood sample and the culture step a0) is a blood culture step.

4. The method according to any one of claims 1 to 3, wherein at step b1) the chromogenic substrate is placed in the presence of at least one β-lactamase inhibitor selected from the group consisting of a cephalosporinase inhibitor, a serine β-lactamase inhibitor, a metal chelator and an extended spectrum β-lactamase inhibitor.

5. The method according to any of claims 1 to 4 wherein the concentration step a1) comprises the centrifugation of the biological sample followed by removal of the supernatant obtained.
